# EUROPEAN PATENT APPLICATION

(11) **EP 2 733 207 A1**
(43) Date of publication of application: **21.05.2014**
(21) Application number: 12811514.4
(22) Date of filing: 12.07.2012
(51) Int. Cl.: C12N 15/09, A61K 35/74, A61K 48/00, A61P 9/10, C12N 1/21

(54) **ISCHEMIC DISEASE THERAPEUTIC AGENT**

(30) Priority: 13.07.2011 US 201161507590 P
(71) Applicant: Anaeropharma Science, Inc., Tokyo 103-0026 (JP)
(72) Inventor: WADA, Yuko, Matsumoto-shi Nagano 390-8621 (JP); SHIMATANI, Yuko, Matsumoto-shi Nagano 390-8621 (JP); SHIMIZU, Hitomi, Matsumoto-shi Nagano 390-8621 (JP); SASAKI, Takayuki, Matsumoto-shi Nagano 390-8621 (JP)
(74) Representative: Zech, Stefan Markus
(86) International application number: PCT/JP2012/067797
(87) International publication number: WO 2013/008881

(57) **Abstract**

[Object]

The purpose of the present invention is to provide: a gene delivery carrier that specifically accumulates only in a lesion site even when systemically administered, has a high rate of protein expression, and disappears from the lesion site as healing occurs; and a therapeutic method for ischemic diseases using same. Said purpose is achieved by providing: a gene delivery carrier comprising an anaerobic bacteria, capable of growing specifically at an ischemic disease site, and capable of expressing at least one protein useful in the diagnosis or treatment of ischemic disease; a medical composition including said gene delivery carrier; and a therapeutic method for ischemic diseases using same.

## Description

### Related Applications

This application is a continuation-in-part of, and claims priority under 35 USC § 120 to, U.S. Serial No. 13/015,806, filed January 28, 2011, which claims priority under 35 USC 119(e) to U.S. Ser. No. 61/299,922 filed January 29, 2010. Further, this application claims priority under 35 USC 119(e) to U.S. Ser. No. 61/507,590 filed July 13, 2011. The disclosures of all of the above are hereby incorporated by reference in their entireties for all purposes.

### [Technical Field]

The present invention relates to a gene transfer carrier and a pharmaceutical composition that can specifically treat the site of an ischemic disease, and a treatment method for an ischemic disease using same.

### [Background Art]

Recently, in the treatment of malignant tumors, a method in which a transformed anaerobic bacterium is used as a gene transfer carrier has been attracting attention; for example, a method in which a gene expressing a nitroreductase, which is an enzyme that converts a prodrug for an antitumor substance into the antitumor substance, is transported to a tumor site using a transformed clostridium has been proposed (ref. Patent Documents 1 to 3).

However, all of the microorganisms that have conventionally been used for the above-mentioned purpose are low toxicity mutants of pathogenic microbes, and the possibility of reverse mutation, with them returning to the original pathogenic microbes and exhibiting toxicity, cannot be ruled out; furthermore, due to mobility and invasiveness there is a possibility that the effect will be exhibited not only in diseased tissue but also in normal tissue to thus cause systemic side effect symptoms, and there is a problem in terms of safety.

Under such circumstances, *Bifidobacterium,* which is a nonpathogenic enterobacterium that is present within and makes up the flora in the human intestine and is known to be a very safe obligately anaerobic bacterium, has been attracting attention, and a transformed *Bifidobacterium* that expresses cytosine deaminase, which is an enzyme that converts 5-fluorocytosine, which is a prodrug for the antitumor substance 5-fluorouracil, into 5-FU has been developed (ref. Patent Documents 4 and 5).

This transformant *Bifidobacterium* has the advantage that when it is intravenously administered into an animal model of solid tumor which is an anaerobic disease, it specifically colonizes and grows in anaerobic diseased tissue in a low oxygen state and quickly disappears in normal tissue that is not in an anaerobic environment (ref. Non-Patent Documents 1 and 2).

In the meantime, in the treatment of an ischemic disease, in particular the treatment of a serious case of ischemia, an angiogenic therapy in which blood flow is restored by regeneration of blood vessels or development of collateral circulation has been attempted. Angiogenic therapy can be broadly divided into three types of therapies, that is, cell transplantation, protein administration, and gene therapy, but from the viewpoint of low invasiveness, gene therapy has particularly been attracting attention in recent years. In angiogenesis by gene therapy, for example, a gene coding for hepatocyte growth factor: HGF, vascular endothelial growth factor: VEGF, etc. is introduced into an area around an affected part by intramuscular injection or intraarterial infusion, thus promoting angiogenesis in the area around the affected part and thereby restoring blood flow (ref. e.g. Non-Patent Documents 3 and 4).

These angiogenesis therapies have been attracting attention as one option for a patient for whom revascularization is not possible or whom the effect is insufficient due to a disorder at the arteriolar level, or a patient who cannot be treated surgically due to a problem with invasiveness and, in particular, with regard to angiogenic therapy by gene therapy, many clinical tests have been carried out in recent years.

### [Prior Art Documents]

### [Patent Documents]

[Patent Document 1] US. Pat. No. 6416754
[Patent Document 2] US. Pat. No. 6652849
[Patent Document 3] US. Pat. Application No. 2003/0103952
[Patent Document 4] JP, A, 2002-97144
[Patent Document 5] WO No. 2007-136107

### [Non-Patent Document]

[Non-Patent Document 1] Yazawa et al., Cancer Gene Therapy, Vol. 7, No. 2, 2000: pp 269-274
[Non-Patent Document 2] Yazawa et al., Breast Cancer Research and Treatment, Vol. 66, 2001: pp 165-170
[Non-Patent Document 3] Gupta et al., Circ. Res. 2009; 105: 724-736
[Non-Patent Document 4] Morishita et al., Arterioscler Thromb Vasc Biol. 2011; 31: 713-720

### [Summary of the Invention]

### [Problems to be Solved by the Invention]

It is an object of the present invention to provide a gene transfer carrier, formed from an anaerobic bacterium, that can grow specifically at the site of an ischemic disease and can express at least one type of protein that is useful for the diagnosis or treatment of an ischemic disease, a pharmaceutical composition containing the gene transfer carrier, and a treatment method for an ischemic disease utilizing same.

### [Means for Solving the Problems]

As hereinabove described, although angiogenic therapy utilizing gene therapy is useful for the treatment of an ischemic disease, current angiogenic therapy utilizing gene therapy still has some problems. Firstly, currently used gene transfer carriers do not have lesion site specificity and when systemically administered they are systemically disseminated, therefore, intramuscular injection or intraarterial infusion is mainly used to administer a transgene; but in these administration methods it cannot be said that it is administered specifically and uniformly to the ischemic disease site and, furthermore, when an ischemic site and a non-ischemic site are present together, a large amount of transgene is delivered to the non-ischemic site, thereby angiogenesis in the non-ischemic site is promoted, and as a result a phenomenon called steal phenomenon in which blood circulation further deteriorates at the ischemic site might occur.

Secondly, since it is a treatment method predicated on a target protein being expressed at a disease site by gene transfection using a vector, it is difficult to control the efficiency of gene transfection or the period of transgene expression. There are therefore the problems that even if administered the transfection is not sufficient thereby an effect is not exhibited, transgene expression stops before remission of the disease, or transgene expression continues after remission.

Thirdly, it is thought that angiogenic therapy is greatly superior to other therapies for the elderly, diabetic patients, etc. because of low invasiveness, but these patients might have complications whose symptoms may be aggravated by angiogenesis, such as for example diabetic retinopathy in a diabetic patient or a malignant tumor in the elderly, and the application of current angiogenic therapy, which has low specificity for a disease site, is limited.

The present inventors have found that all of these problems can be solved by a vector that specifically accumulates only at a disease site even with systemic administration, has a high protein expression rate, and disappears from the disease site accompanying a cure, and as a result of an intensive investigation the present invention has been accomplished.

That is, the present invention relates to the following.
(1) A gene transfer carrier consisted of an anaerobic bacterium that can grow specifically at the site of an ischemic disease and can express at least one type of protein that is useful for the diagnosis or treatment of an ischemic disease.
(2) The gene transfer carrier according to (1), wherein the anaerobic bacterium is transformed using a transforming plasmid, said plasmid comprising a DNA sequence coding for the protein that is useful for the diagnosis or treatment of an ischemic disease.
(3) The gene transfer carrier according to (1) or (2), wherein the ischemic disease is a chronic ischemic disease.
(4) The gene transfer carrier according to (1) to (3), wherein the anaerobic bacterium is a nonpathogenic enterobacterium.
(5) The gene transfer carrier according to (1) to (4), wherein the anaerobic bacterium is a *Bifidobacterium.*
(6) The gene transfer carrier according to (5), wherein the *Bifidobacterium* is one type selected from the group consisting of *Bifidobacterium adolescentis, Bifidobacterium angulatum, Bifidobacterium animalis, Bifidobacterium asteroides, Bifidobacterium bifidum, Bifidobacterium boum, Bifidobacterium breve, Bifidobacterium catenulatum, Bifidobacterium choerinum, Bifidobacterium coryneforme, Bifidobacterium cuniculi, Bifidobacterium denticolens, Bifidobacterium dentium, Bifidobacterium gallicum, Bifidobacterium gallinarum, Bifidobacterium globosum, Bifidobacterium indicum, Bifidobacterium infantis, Bifidobacterium inopinatum, Bifidobacterium lactis, Bifidobacterium lactentis, Bifidobacterium liberorum, Bifidobacterium longum, Bifidobacterium magnum, Bifidobacterium merycicum, Bifidobacterium minimum, Bifidobacterium mongoliens, Bifidobacterium parvulorum, Bifidobacterium pseudocatenulatum, Bifidobacterium pseudolongum, Bifidobacterium psychraerophilum, Bifidobacterium pullorum, Bifidobacterium ruminale, Bifidobacterium ruminantium, Bifidobacterium saeculare, Bifidobacterium scardovi, Bifidobacterium subtile, Bifidobacterium suis, Bifidobacterium thermacidophillum,* and *Bifidobacterium thermophilum.*
(7) The gene transfer carrier according to (6), wherein the *Bifidobacterium* is *Bifidobacterium longum.*
(8) The gene transfer carrier according to (1) to (7), wherein the protein that is useful for the diagnosis of an ischemic disease is a fluorescent protein.
(9) The gene transfer carrier according to (1) to (7), wherein the protein that is useful for the treatment of an ischemic disease is at least one type selected from the group consisting of fibroblast growth factor (FGF), endothelial cell growth factor (ECGF), vascular endothelium growth factor (VEGF), hepatocyte growth factor (HGF), angiogenic growth factor (AGF), platelet-derived growth factor (PDGF), transforming growth factor β (TGFβ), a protein having angiogenesis promoting activity such as angiopoietin or ephrin, a factor involved in vasodilation such as a prostaglandin, a colony stimulating factor such as granulocyte colony stimulating factor (G-CSF) or granulocyte-macrophage colony stimulating factor (GM-CSF), nerve growth factor (NGF), brain-derived neurotrophic factor (BDNF), a neurotrophin such as neurotrophin 3, and insulin-like growth factor (IGF).
(10) The gene transfer carrier according to (2) to (9), wherein the transforming plasmid is a non-shuttle plasmid.
(11) The gene transfer carrier according to (2) to (10), wherein the transforming plasmid further comprises a gene sequence coding for a secretory signal peptide.
(12) The gene transfer carrier according to any one of (2) to (11), wherein the transforming plasmid comprises pTB6 rep unit.
(13) The gene transfer carrier according to any one of (2) to (12), wherein the transforming plasmid has an expression cassette comprising p37 promoter, HU terminator and a gene coding for FGF2.
(14) The gene transfer carrier according to any one of (2) to (13), wherein the transforming plasmid comprises a DNA sequence coding for a polypeptide having the sequence described in SEQ ID No: 40.
(15) The gene transfer carrier according to any one of (2) to (14), wherein the transforming plasmid is pFGF12a (SEQ ID No: 38).
(16) A pharmaceutical composition for an ischemic disease comprising the gene transfer carrier according to (1) to (15).
(17) The pharmaceutical composition according to (16), wherein it is administered by systemic administration.
(18) A method for diagnosing or treating an ischemic disease, the method comprising administering an anaerobic bacterium that can specifically grow at the site of an ischemic disease and can express at least one type of protein that is useful for the diagnosis or treatment of an ischemic disease.
(19) The method according to (18), wherein said administering is systemically administering.

### [Effects of the Invention]

Since the gene transfer carrier of the present invention is an anaerobic bacterium, it colonizes and grows specifically at the site of an ischemic disease, which is under an anaerobic environment, can produce and secrete a protein having therapeutic activity for an ischemic disease in the diseased tissue, is very useful as a drug for the treatment of an ischemic disease, and can be expected to be a high quality gene transfer carrier. Furthermore, since it colonizes specifically at the site of an ischemic disease, it specifically accumulates at the site of an ischemic disease and exhibits an effect even with systemic administration such as intravenous injection. Therefore, there is no necessity for the administration of a large amount or administration multiple times, and the burden on the administration subject can be alleviated. Moreover, since the anaerobic environment at the site of the ischemic disease is maintained while the ischemia continues, it grows for a long period of time, but once the ischemia enters remission, the anaerobic environment is no longer maintained, it cannot grow and quickly disappears.

Furthermore, since the gene transfer carrier of the present invention can itself express a protein that is useful for treatment, it is not necessary to take into consideration the efficiency of gene transfer to the ischemic site or the cells in its vicinity as in the conventional art, and high protein expression efficiency can always be exhibited. Moreover, since it is a carrier that is specifically delivered to the ischemic site, there are no concerns about complications. It is therefore possible to carry out an angiogenic treatment that is less invasive than the conventional art, causes fewer side effects, and has high safety. Furthermore, making the gene transfer carrier of the present invention simultaneously express a marker enables it to be used for the diagnosis of an ischemic site or as a monitor for a treatment. Moreover, the gene carrier of the present invention can deliver a plurality of genes at the same time, and it is thought that a more efficient and noninvasive treatment will become possible by incorporating a plurality of effective growth factors and administering.

### [Brief Description of Drawings]

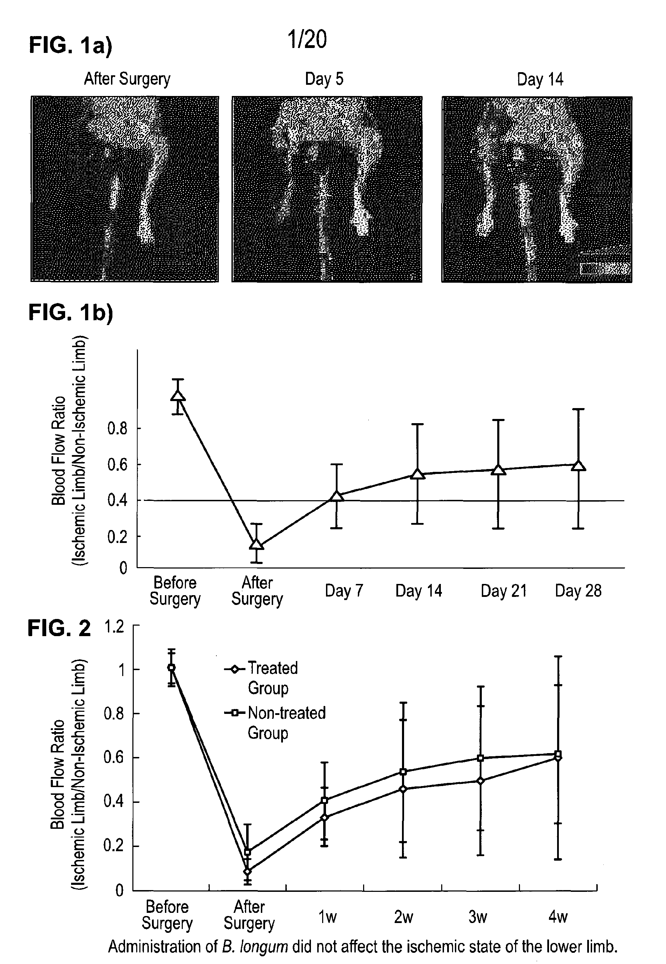

[FIG. 1] Figure 1 shows: a) images of measurement by a laser Doppler blood flow meter of a lower limb ischemia model mouse (ischemic model), and b) a graph showing change in blood flow ratio (ischemic limb/non-ischemic limb).
[FIG. 2] Figure 2 is a graph comparing the change in blood flow ratio between the group that was treated with *B. longum* of the present invention and the group that was not treated in the ischemic model. There was no significant difference in the change in blood flow ratio between the treated group and the non-treated group.

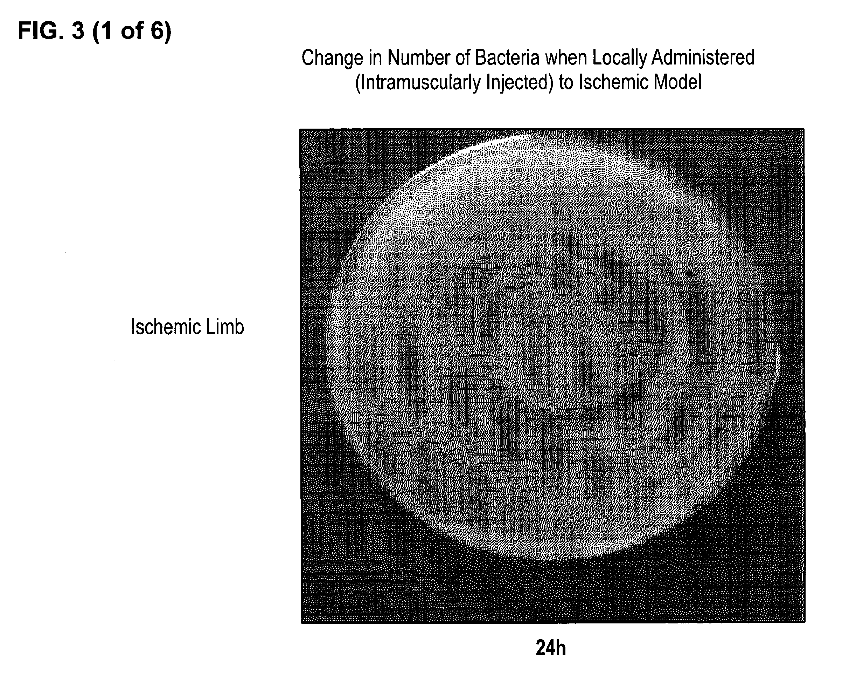

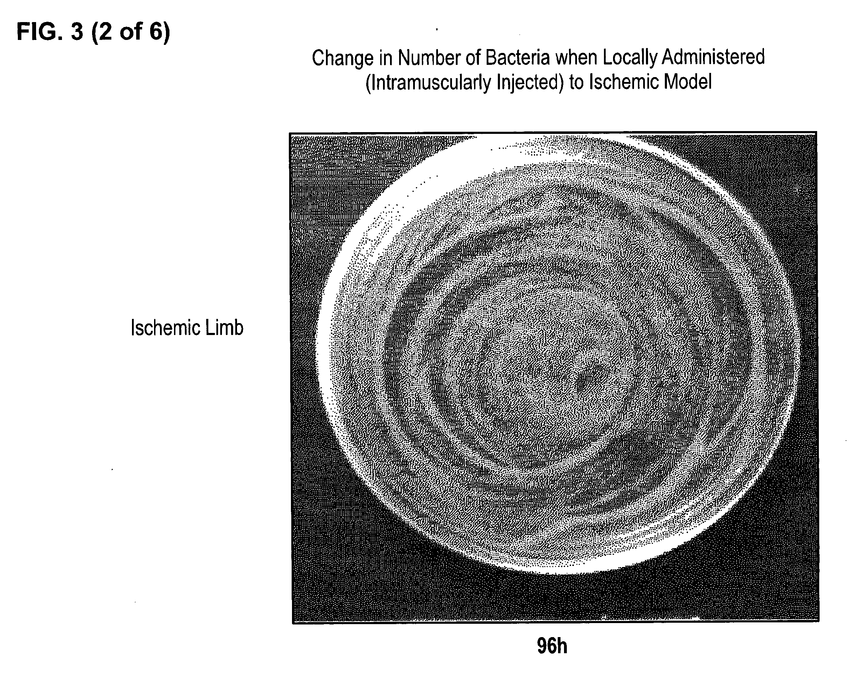

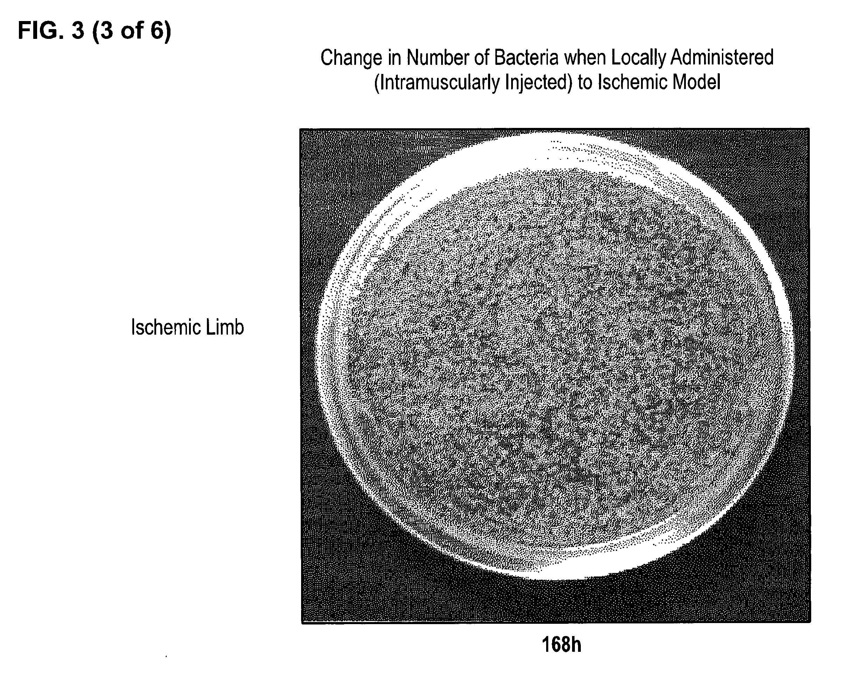

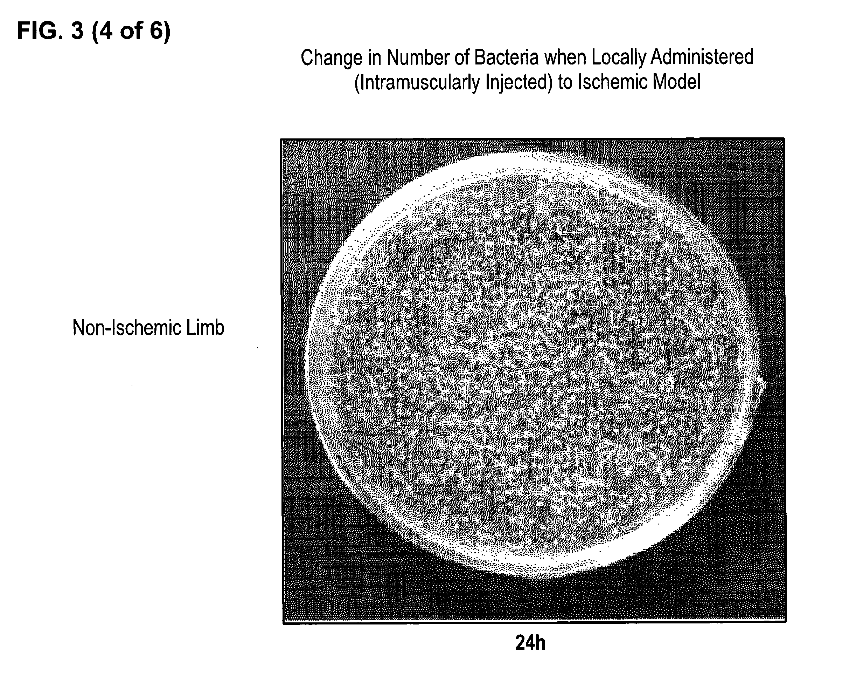

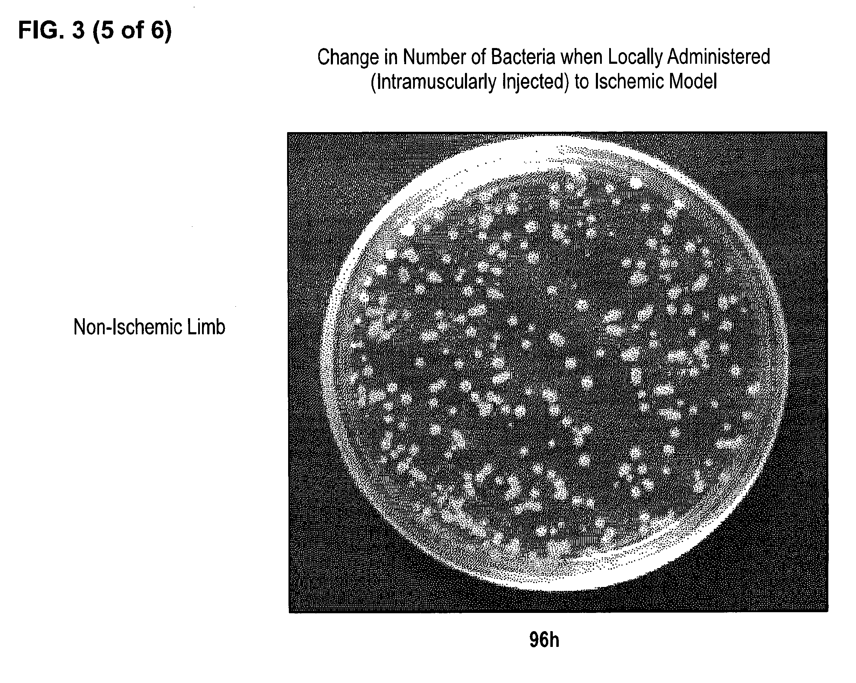

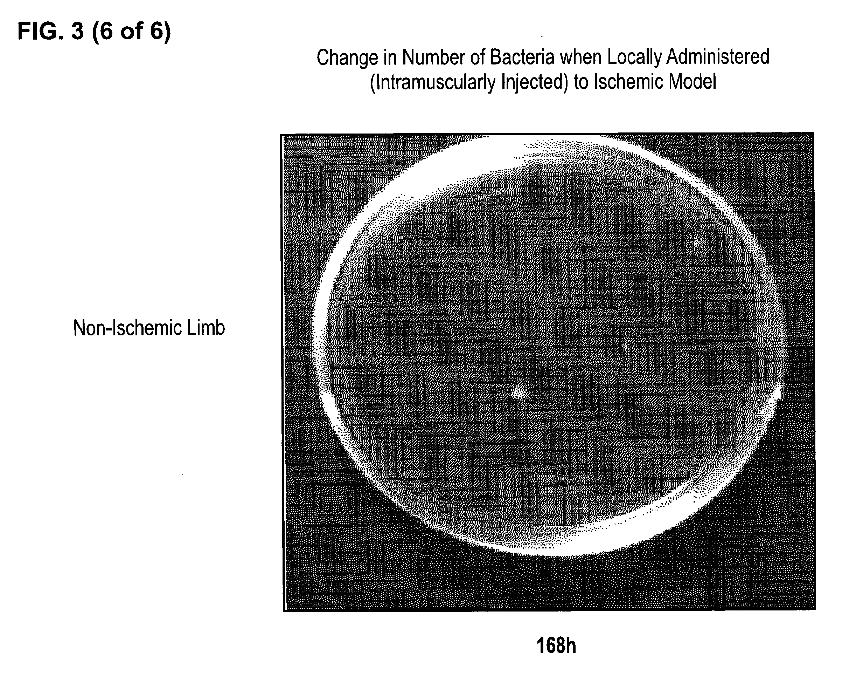

[FIG. 3] Figure 3 shows photographs of detected *B. longum* of the present invention existing in samples collected from an ischemic limb and a non-ischemic limb of the ischemic model local administration group. In the ischemic limb, a large number of bacteria were observed even at 168 hours after administration, but in the non-ischemic limb almost none were observed.

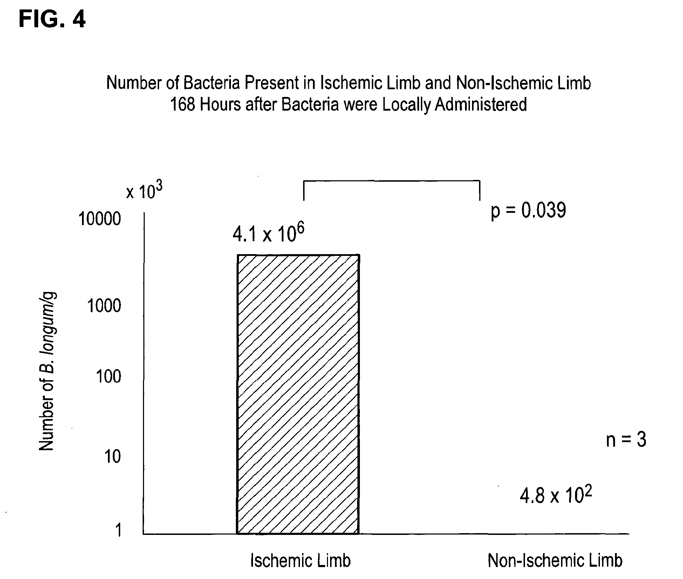

[FIG. 4] Figure 4 is a graph of bacterial count in the ischemic limb and the non-ischemic limb at 168 hours after administration to the ischemic model local administration group.

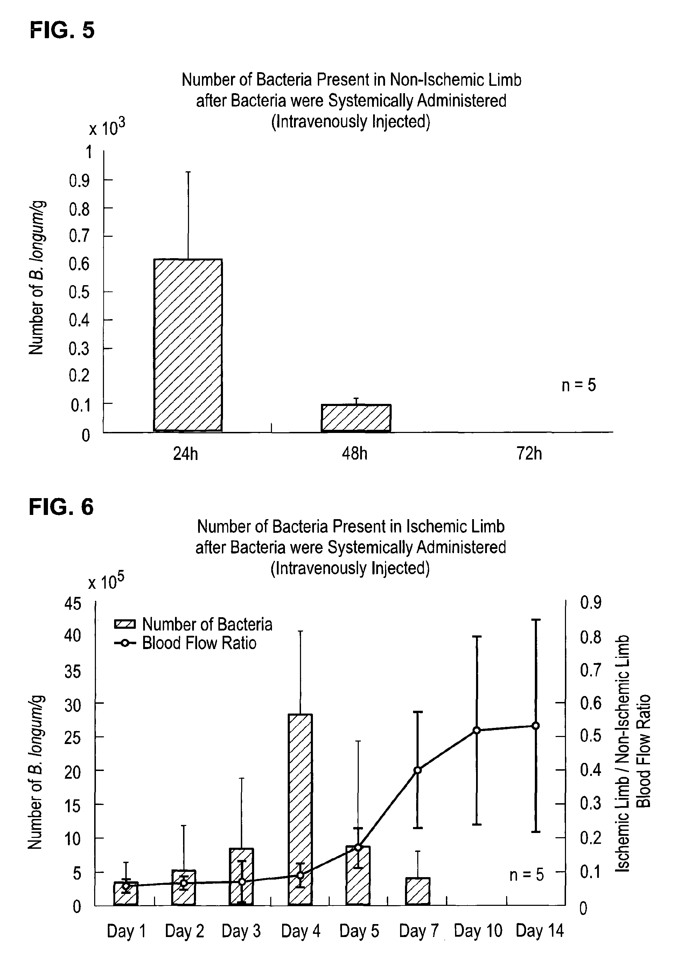

[FIG. 5] Figure 5 is a graph of the change in *B. longum* count at 24 hours, 48 hours and 72 hours in the non-ischemic limb of the ischemic model systemic administration group. Even in the systemic administration group almost no bacteria were observed at 48 hours, and none were observed at 72 hours.
[FIG. 6] Figure 6 is a graph of change in blood flow ratio and change in bacterial count with respect to time in the ischemic limb of the ischemic model systemic administration group. It can be seen that the bacterial count increased with time after administration, and the bacterial count decreased as the blood flow to the ischemic site recovered.

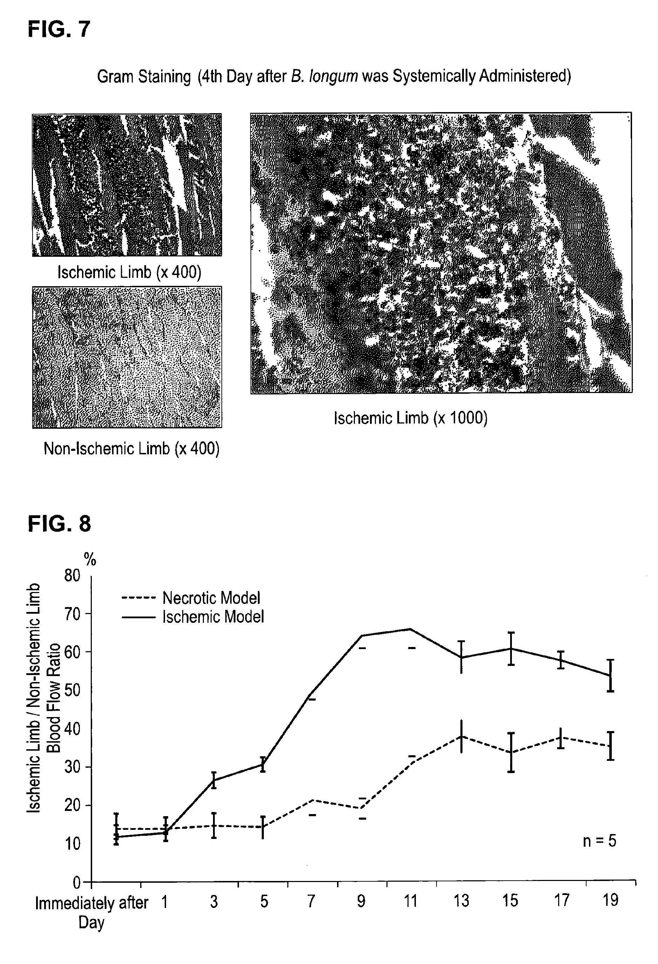

[FIG. 7] Figure 7 shows Gram stain images of muscle tissue at 4 days after administration in the ischemic model systemic administration group. Whereas no Gram-stained bacteria were observed in the non-ischemic limb, many Gram-stained bacteria were observed in the ischemic limb.
[FIG. 8] Figure 8 is a graph of change over time of ischemic limb/non-ischemic limb blood flow ratio of the lower limb ischemia model mouse (ischemic model) and the lower limb necrosis model mouse (necrotic model). It can be seen that in the necrotic model the ischemic state was of longer duration and the degree of recovery was low.

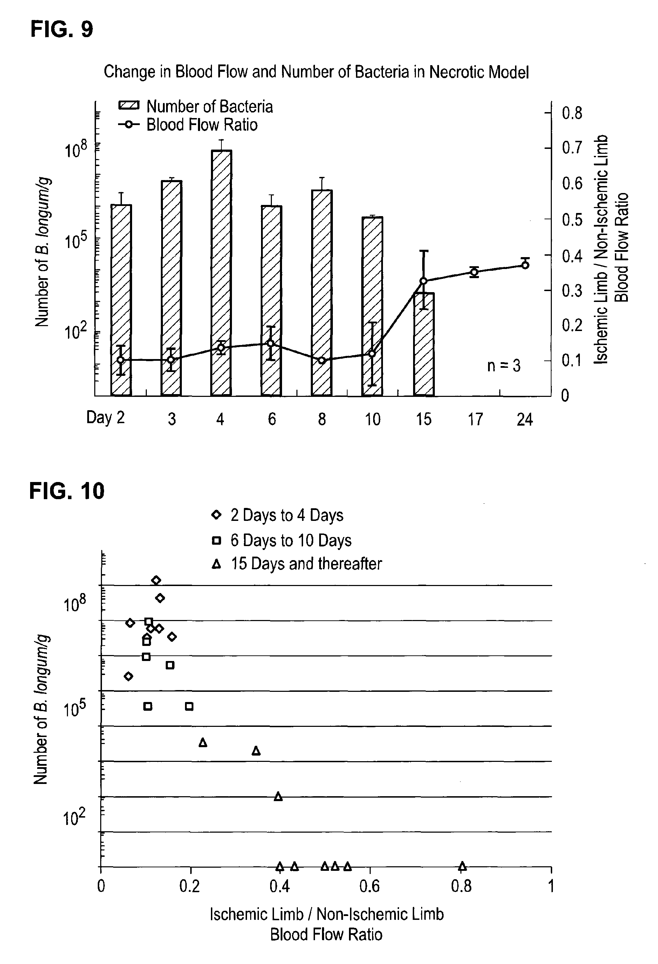

[FIG. 9] Figure 9 is a graph of change in blood flow ratio and change in bacterial count with respect to time in the ischemic limb of the necrotic model systemic administration group. It can be seen that the bacterial count increased with time after administration, *B. longum* colonized and grew while the ischemic state continued, and the bacterial count decreased as the blood flow to the ischemic site recovered.
[FIG. 10] Figure 10 is a dot diagram of *B. longum* bacterial count plotted with respect to ischemic limb/non-ischemic limb blood flow ratio in the ischemic limb of the necrotic model systemic administration group. It can be seen that the bacterial count decreased as the blood flow ratio increased.

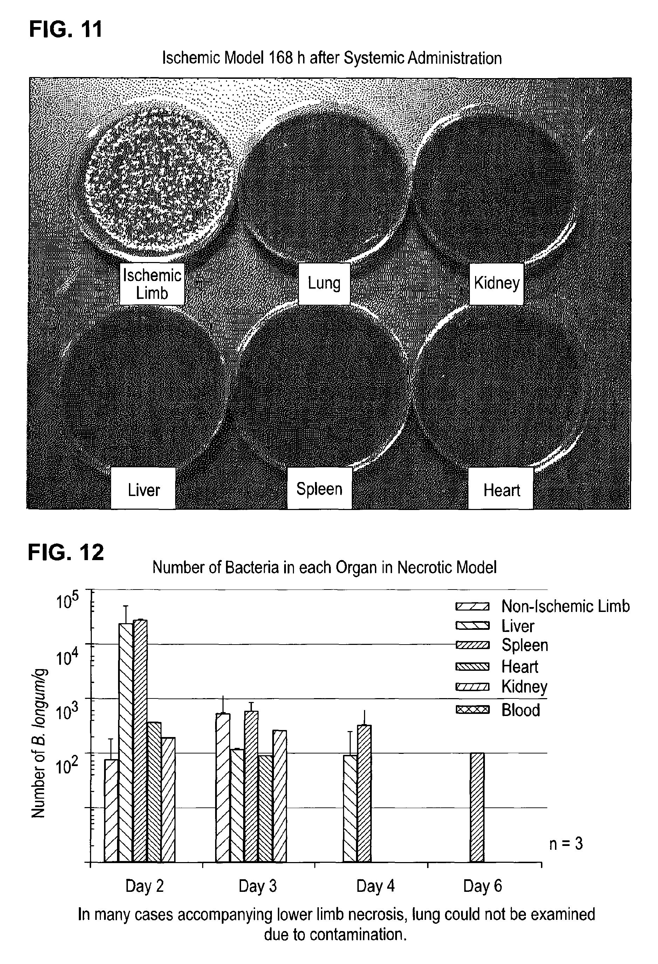

[FIG. 11] Figure 11 is a photograph of the results of a B. *longum* detection test in the ischemic limb, the lung, the kidney, the liver, the spleen, and the heart of the ischemic model systemic administration group. Upper left is ischemic limb, upper middle is lung, upper right is kidney, lower left is liver, lower middle is spleen, and lower left is heart. Even with systemic administration, the administered *B. longum* was not observed in organs other than the ischemic limb, which was the ischemic disease site.
[FIG. 12] Figure 12 is a graph of *B. longum* bacterial count with time elapsed in the non-ischemic limb, the blood, the kidney, the liver, the spleen, and the heart of the necrotic model systemic administration group. It can be seen that some bacteria were observed immediately after administration, but they quickly disappeared thereafter. Bacteria were not detected in the blood.

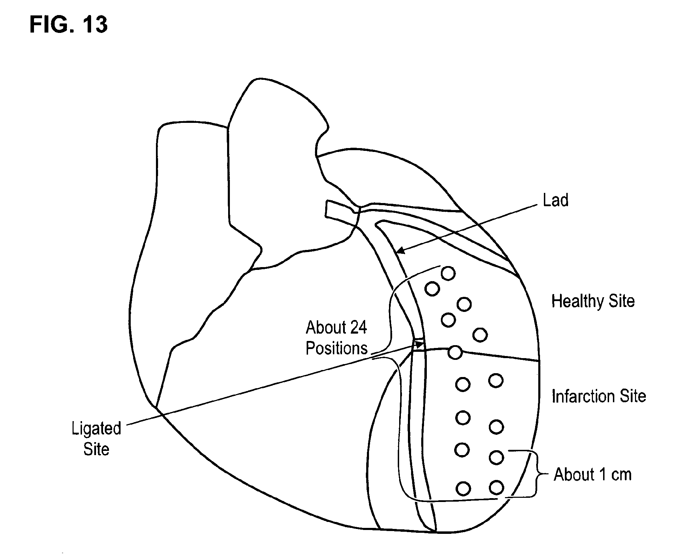

[FIG. 13] Figure 13 is a schematic diagram showing the ligation site and the location of bacterial administration of the myocardial infarction model.

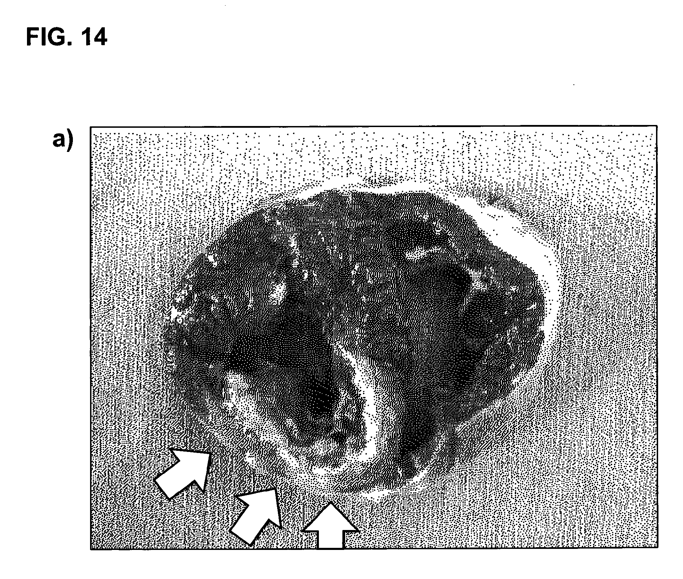

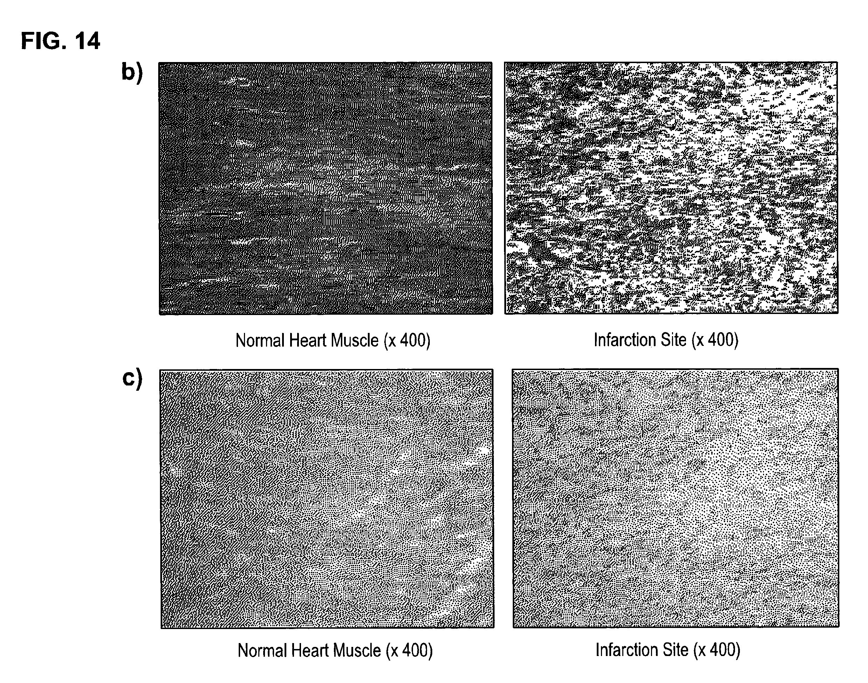

[FIG. 14] Figure 14 shows stain images of the heart of the miniature swine myocardial infarction model. a) is a TTC stain image, in which the infarction site (indicated by arrows) was not stained, showing that it is an infarction site. b) and c) are an MT stain image and an HE stain image at the normal myocardial site and the infarction site, respectively. In both of the stain images, depletion of myocardial cells and progression of fibrosis were observed at the infarction site.

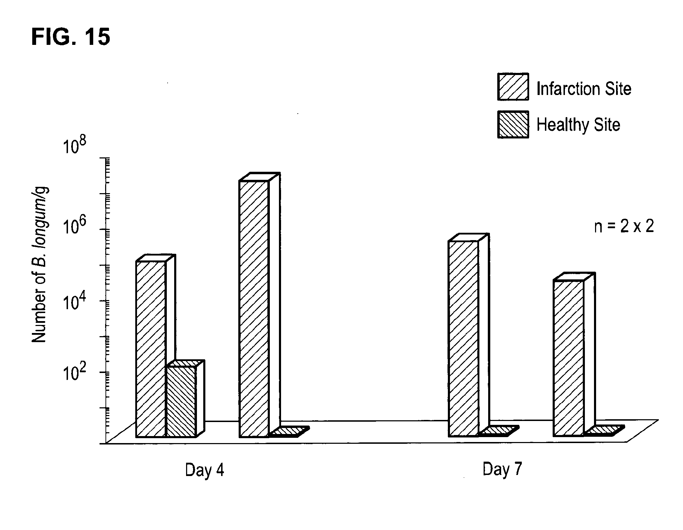

[FIG. 15] Figure 15 is a graph showing *B. longum* bacterial count at the infarction site and the healthy site at 4 days and 7 days after administration of *B. longum* in the myocardial infarction model. At 4 days after administration a few bacteria were also observed at the healthy site, but at day 7 bacteria were observed only at the infarction site.

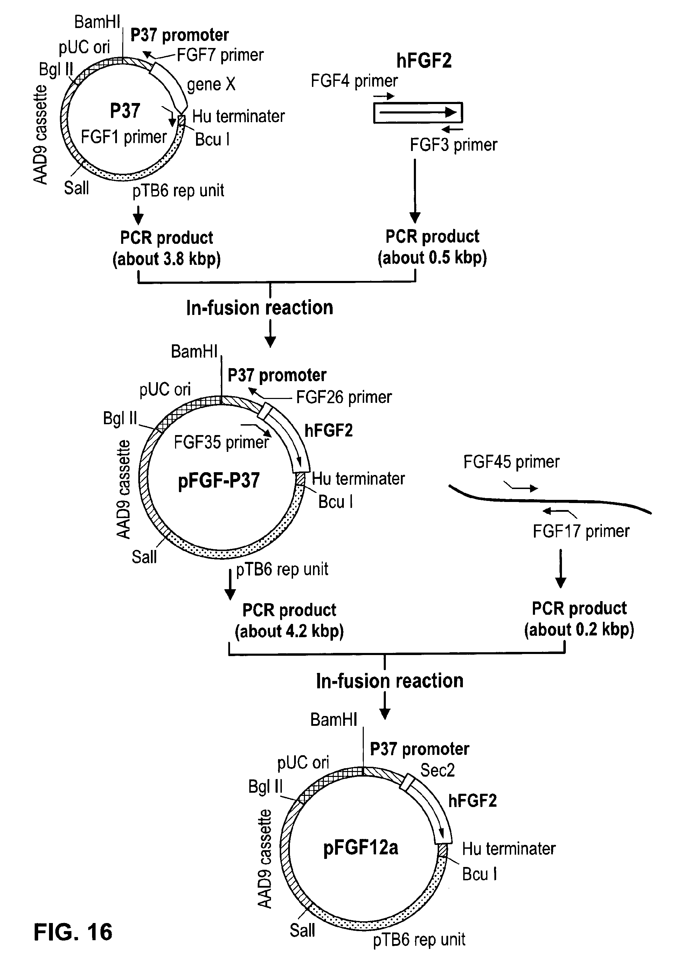

[Fig. 16] Fig. 16 is a diagram showing a scheme for constructing pFGF12a, which is one embodiment of the transforming plasmid that transforms the gene transfer carrier of the present invention.

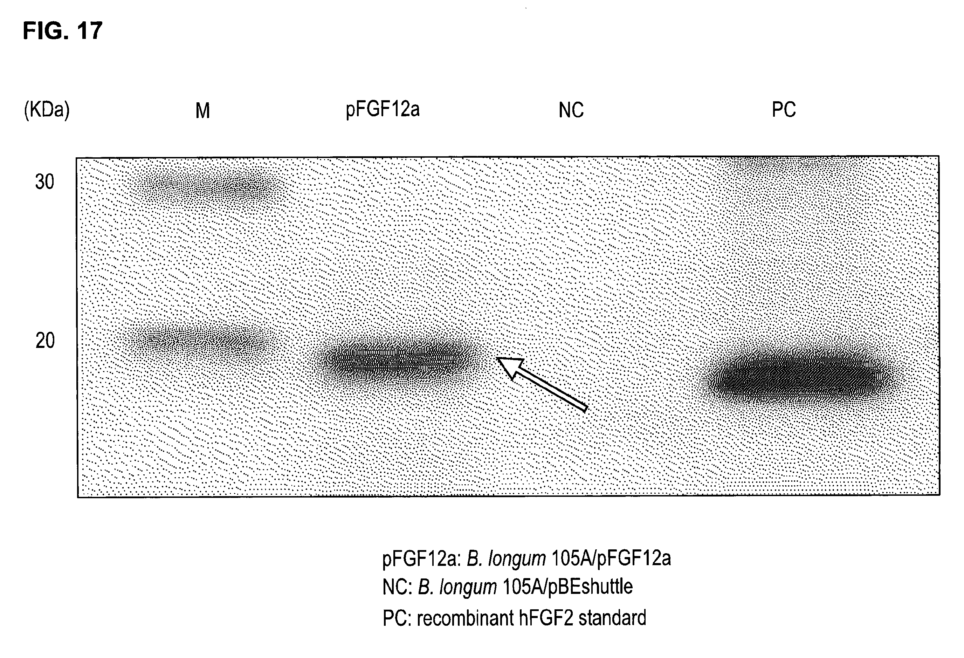

[Fig. 17] Fig. 17 shows the results of Western blotting using culture supernatant of Bifidobacterium longum 105A that has been transformed with pFGF12a. Bifidobacterium longum 105A transformed with pBEshuttle is used as a negative control, and hFGF2 is used as a positive control. A band of approximately 20kDa was confirmed, being considered to be hFGF2.

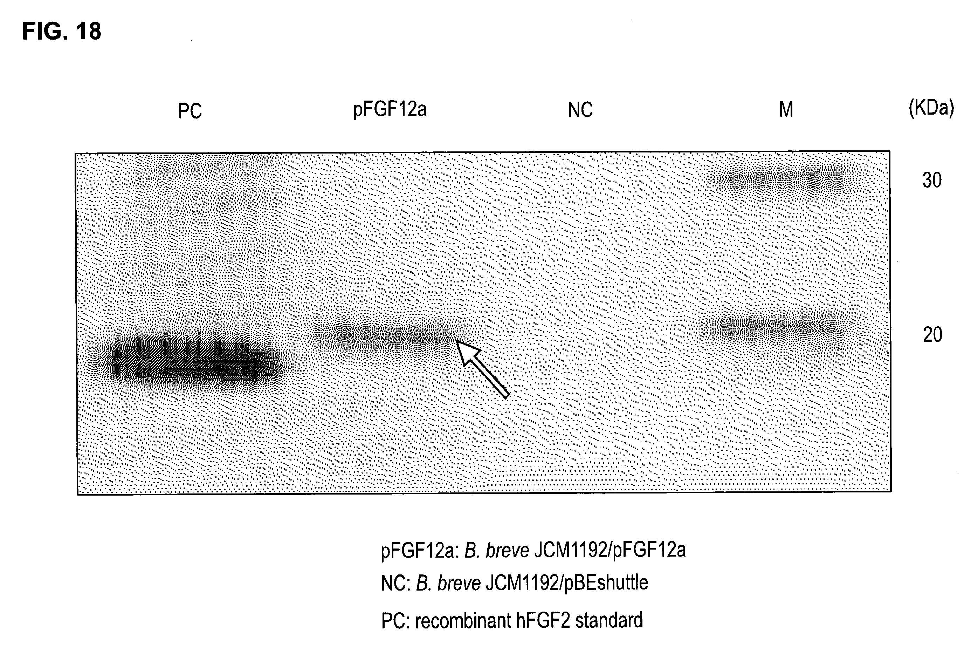

[Fig. 18] Fig. 18 shows the results of Western blotting using culture supernatant of Bifidobacterium breve JCM1192 that has been transformed with pFGF12a. Bifidobacterium breve JCM1192 transformed with pBEshuttle is used as a negative control, and hFGF2 is used as a positive control. Again, a band of approximately 20kDa was confirmed, being considered to be hFGF2.

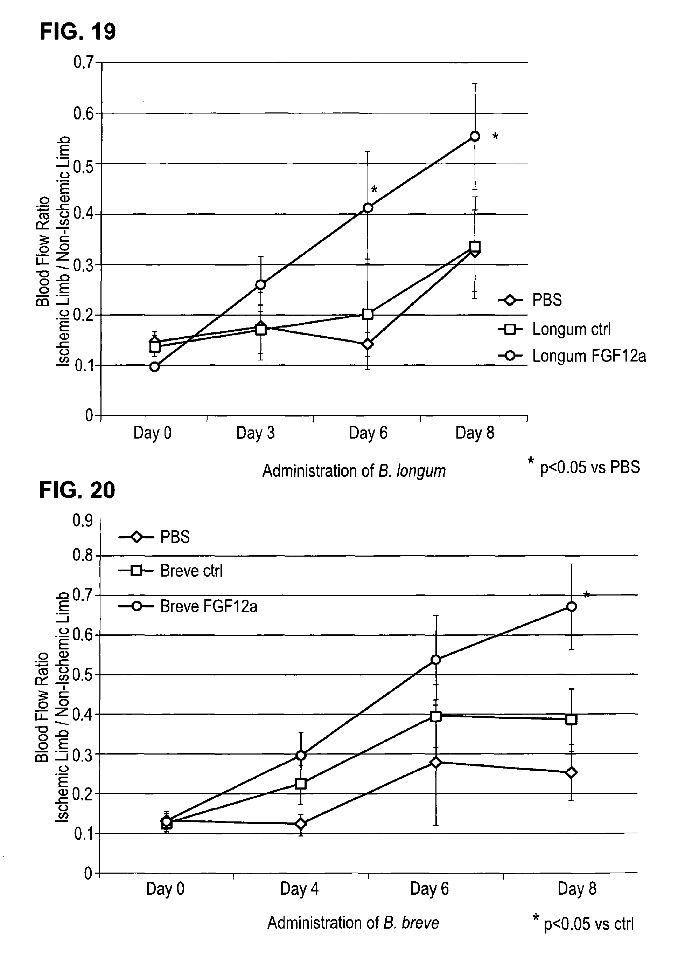

[Fig. 19] Fig. 19 is a graph showing changes in blood flow in lower limb ischemic site of ischemic model mice which had been given an intravenous injection of the pharmaceutical composition comprising Bifidobacterium longum 105A/pFGF12a, i.e., the gene transfer carrier of the invention. Though there was already a small difference in the recovery of blood flow on Day 3, a significant recovery of blood flow was observed after Day 6 in the group which had been treated with the gene transfer carrier of the invention.
[Fig. 20] Fig. 20 is a graph showing changes in blood flow in lower limb ischemic site of ischemic model mice which had been given an intravenous injection of the pharmaceutical composition comprising Bifidobacterium breve JCM1192/pFGF12a, i.e., the gene transfer carrier of the invention. Though there was already a small difference in the recovery of blood flow on Day 4, a significant recovery of blood flow was observed after Day 8 in the group which had been treated with the gene transfer carrier of the invention.

### [Modes for Carrying Out the Invention]

The present invention relates to a gene transfer carrier formed from an anaerobic bacterium that can grow specifically at the site of an ischemic disease and can express at least one type of protein that is useful for the diagnosis or treatment of an ischemic disease.

In the present invention, 'ischemia' means a state in which there is a shortage of oxygen and nutrients in tissue due to a decrease in the amount of arterial blood supplied to the tissue caused by constriction or blockage of blood vessels, and persistent ischemia causes tissue atrophy, degeneration, necrosis, etc.

The gene transfer carrier of the present invention is mainly used in a treatment method for improving an undesirable state caused by ischemia, such as an angiogenic treatment or protection of an organ. Therefore, in the present specification, an 'ischemic disease' is a state in which, regardless of the presence or absence of subjective symptoms, ischemia of the tissue is sustained by the constriction or blockage of arteries or an undesirable state caused by such ischemia. Examples of the ischemic disease include, but are not limited to, an ischemic heart disease such as angina pectoris or myocardial infarction, a cerebral ischemia such as cerebral infarction, a chronic cerebral ischemia such as moyamoya disease, spinal ischemia, an ischemic bowel disease such as ischemic colitis or mesenteric arterial occlusion, a lower limb ischemia such as arteriosclerosis obliterans, and a retinal ischemia such as diabetic retinopathy.

In the present specification, 'ischemic site' means a site in a state in which there is a shortage of arterial blood flow, nutrients, and oxygen due to ischemia, and is used interchangeably with 'site of an ischemic disease' or 'ischemic diseased tissue'.

In the present specification, 'anaerobic bacterium' means a bacterium having anaerobic properties, and 'anaerobic properties' means the property of being capable of growing under conditions where there is little or no oxygen. Anaerobic bacteria can generally be classified into facultative anaerobic bacteria, which can grow in the presence of oxygen, and obligately anaerobic bacteria, which cannot grow in the presence of oxygen, and in the present invention obligately anaerobic bacteria are preferable. The anaerobic properties of the anaerobic bacterium of the present invention may be properties that the bacterium has intrinsically or those obtained by transformation.

Since the gene transfer carrier of the present invention is formed from an anaerobic bacterium, it can colonize and grow specifically at the site of an ischemic disease, which is under anaerobic conditions. Since the gene transfer carrier itself has gene transcription and translation functions, it can express a protein that is useful for diagnosis or treatment when it colonizes the ischemic diseased tissue, and can supply it to the diseased tissue. Therefore, the gene transfer carrier of the present invention comprises a DNA sequence coding for a protein that is useful for the diagnosis or treatment of an ischemic disease.

In one embodiment of the present invention, the gene transfer carrier is transformed by a transforming plasmid. Any transforming plasmid may be used as long as it functions in the gene transfer carrier bacterium and does not impair the anaerobic properties of the bacterium.

As described above, the anaerobic properties of the anaerobic bacterium that is the gene transfer carrier of the present invention may be those obtained by transformation, and in one embodiment of the present invention the anaerobic bacterium is transformed so as to be an obligately anaerobic bacterium.

In a preferred embodiment of the present invention, the gene transfer carrier is transformed by a plasmid having a DNA sequence coding for a protein that is useful for the diagnosis or treatment of an ischemic disease. Imparting by transformation an ability to express a protein that is useful for the diagnosis or treatment of an ischemic disease enables any protein to be expressed in accordance with the design of a plasmid.

In the present invention, the ischemic disease includes an acute ischemic disease in which tissue is damaged as a result of a rapid decrease of the oxygen concentration caused by ischemia, and a chronic ischemic disease in which denaturing or necrosis of tissue is caused by a low oxygen concentration sustained for a long period of time. Since the gene transfer carrier of the present invention is selectively delivered to a disease site after being administered, colonizes and remains at the delivery site, and exhibits an effect, it is preferably used for improvement of a chronic ischemic state. In the present specification, the term 'chronic ischemic state' is used interchangeably with the term 'chronic ischemic disease', and examples include, but are not limited to, an ischemic heart disease such as angina pectoris or myocardial infarction, a chronic cerebral ischemia such as moyamoya disease, and a lower limb ischemia such as arteriosclerosis obliterans. Examples of a treatment for improving a chronic ischemic disease include, but are not limited to, an angiogenic therapy.

Since it is assumed that the gene transfer carrier of the present invention is administered internally, it is necessary for the anaerobic bacterium that is used to have no toxicity or little toxicity. Therefore, in one embodiment of the present invention, the gene transfer carrier can be a mutant that is formed by mutating a pathogenic bacterium so that it has low toxicity. However, there is a possibility that a mutant bacterium having low toxicity will return to the original pathogenic microbe by reverse mutation and exhibit toxicity, and it is therefore preferable to use an intrinsically nonpathogenic bacterium. Therefore, in a preferred embodiment of the present invention, the anaerobic bacterium used is a nonpathogenic enterobacterium.

As the nonpathogenic enterobacterium that can be used in the present invention, a bacterium belonging to the *Bifidobacterium* genus (*Bifidobacterium*) can be cited. Examples of bacteria belonging to the *Bifidobacterium* genus include *Bifidobacterium adolescentis, Bifidobacterium angulatum, Bifidobacterium animalis, Bifidobacterium asteroides, Bifidobacterium bifidum, Bifidobacterium boum, Bifidobacterium breve, Bifidobacterium catenulatum, Bifidobacterium choerinum, Bifidobacterium coryneforme, Bifidobacterium cuniculi, Bifidobacterium denticolens, Bifidobacterium dentium, Bifidobacterium gallicum, Bifidobacterium gallinarum, Bifidobacterium globosum, Bifidobacterium indicum, Bifidobacterium infantis, Bifidobacterium inopinatum, Bifidobacterium lactis, Bifidobacterium lactentis, Bifidobacterium liberorum, Bifidobacterium longum, Bifidobacterium magnum, Bifidobacterium merycicum, Bifidobacterium minimum, Bifidobacterium mongoliens, Bifidobacterium parvulorum, Bifidobacterium pseudocatenulatum, Bifidobacterium pseudolongum, Bifidobacterium psychraerophilum, Bifidobacterium pullorum, Bifidobacterium ruminale, Bifidobacterium ruminantium, Bifidobacterium saeculare, Bifidobacterium scardovi, Bifidobacterium subtile, Bifidobacterium suis, Bifidobacterium thermacidophillum,* and *Bifidobacterium thermophilum,* and *Bifidobacterium longum* is most preferable.

All of these bacteria are commercially available or can be obtained easily from a depository. For example, *Bifidobacterium longum* ATCC-15707, *Bifidobacterium bifidum* ATCC-11863, *Bifidobacterium infantis* ATCC-15697, etc. can be obtained easily from ATCC (The American Type Culture Collection).

Furthermore, the strain of each bacterium is not particularly limited; examples of strains of *Bifidobacterium longum* include *Bifidobacterium longum* 105-A strain, *Bifidobacterium longum* aE-194b strain, *Bifidobacterium longum* bs-601 strain, and *Bifidobacterium longum* M101-2 strain, and among them *Bifidobacterium longum* 105-A strain is preferable.

Examples of strains of *Bifidobacterium breve* include *Bifidobacterium breve* standard strain (JCM1192), *Bifidobacterium breve* aS-1 strain, and *Bifidobacterium breve* I-53-8W strain, and among them *Bifidobacterium breve* standard strain and *Bifidobacterium breve* aS-1 strain are preferable.

Examples of strains of *Bifidobacterium infantis* include *Bifidobacterium infantis* standard strain (JCM1222) and *Bifidobacterium infantis* I-10-5 strain, and among them *Bifidobacterium infantis* standard strain and *Bifidobacterium infantis* I-10-5 strain are preferable.

Examples of strains of *Bifidobacterium lactentis* include *Bifidobacterium lactentis* standard strain (JCM1220).

Since the gene transfer carrier of the present invention colonizes specifically at the site of an ischemic disease, it is possible to diagnose the site of an ischemic disease by detecting the presence of the gene transfer carrier. Detection of the gene transfer carrier can be carried out simply by for example labeling the gene transfer carrier. From the viewpoint of use in the diagnosis of a disease, it is preferable to carry out detection with low invasiveness, and it is preferable that there is little adverse effect on a delivery target by labeling. Therefore, in a preferred embodiment of the present invention, the gene transfer carrier expresses a fluorescent protein as a protein that is useful for diagnosis. Examples of the fluorescent protein include various types of green fluorescent proteins (GFPs) and red fluorescent proteins (RFPs).

Since the gene transfer carrier of the present invention colonizes specifically at the site of an ischemic disease, a protein that is expressed at the colonization site is inevitably delivered specifically to the site of the ischemic disease. Therefore, making the gene transfer carrier of the present invention express a protein that is used for the treatment of an ischemic disease enables the ischemic disease to be treated effectively. Therefore, in a preferred embodiment of the present invention, the gene transfer carrier expresses a protein that is useful for the treatment of an ischemic disease.

Examples of the protein that is useful for the treatment of an ischemic disease include, but are not limited to, a protein having angiogenesis promoting activity and a protein involved in vasodilation. Examples of proteins having angiogenesis promoting activity include, but are not limited to, fibroblast growth factor (FGF), endothelial cell growth factor (ECGF), vascular endothelium growth factor (VEGF), hepatocyte growth factor (HGF), angiogenic growth factor (AGF), platelet-derived growth factor (PDGF), transforming growth factors β (TGF β), angiopoietin, and ephrin, and examples of factors involved in vasodilation include a prostaglandin. Examples of other proteins that are useful for the treatment include colony stimulating factors such as granulocyte colony stimulating factor (G-CSF) and granulocyte-macrophage colony stimulating factor (GM-CSF), nerve growth factor (NGF), brain-derived neurotrophic factor (BDNF), a neurotrophin such as neurotrophin 3, and insulin-like growth factor (IGF).

With regard to a plasmid that can be used in transformation of the gene transfer carrier of the present invention, any plasmid may be used as long as it functions in the gene transfer carrier bacterium and does not impair the anaerobic properties of the bacterium as described above. However, there is a possibility that, in the body of the delivery target, a plasmid that is used for transformation might be horizontally transferred to another bacterium within the body such as for example E. coli, and in this case there is an undeniable risk that the plasmid will replicate in the other bacterium to which it is horizontally transferred, and as a result the protein encoded by the plasmid will be expressed at an unintended site. Therefore, in a preferred embodiment, the transforming plasmid is a non-shuttle plasmid.

In the present specification, the term 'shuttle plasmid' means a plasmid that can replicate in two or more different hosts, and is used interchangeably with the term 'shuttle vector plasmid'. Therefore, the term 'non-shuttle plasmid' means a plasmid that can replicate only in one type of host.

The gene transfer carrier of the present invention produces a protein that is encoded by the transforming plasmid in the bacterial body, and since such a protein exhibits its therapeutic effect only when it is released from the bacterial body, in order to make a protein that is usually not secreted from the bacterial body exhibit a therapeutic effect, it is necessary to destroy the bacterium. In order to solve this problem, in a preferred embodiment the transforming plasmid further comprises a gene sequence coding for a secretory signal peptide.

In the present specification, the term 'secretory signal peptide' means a peptide sequence, present at the terminal of a protein, having the function of secreting a protein produced within the bacterial body from the bacterial body. Examples of secretory signal peptides that can be used include, but are not limited to, amyB of *Bifidobacterium adolescentis,* Sec1, Sec2, and Sec3 of *Bifidobacterium breve,* and peptides encoded by DNA sequences of SEQ ID Nos: 1 to 22.

The gene transfer carrier of the present invention may be for example prepared as described below.

For example, in accordance with a standard method, a gene coding for at least one type of a protein that is useful for the diagnosis or treatment of a desired ischemic disease is inserted into a shuttle plasmid having a replication initiation site that functions in both a transformed bacterium and a bacterium other than the transformed bacterium, for example, *Bifidobacterium* and E. coli, thus preparing a shuttle plasmid.

If desired, removing the replication initiation site for the bacterium other than the transformed bacterium from this shuttle plasmid enables a non-shuttle plasmid to be prepared.

Furthermore, if desired, replacing a promoter gene with a secretory signal and its promoter gene functioning in at least *Bifidobacterium,* and replacing a terminator gene with a terminator gene of the secretory signal peptide functioning in *Bifidobacterium* enables a plasmid comprising a gene sequence coding for the secretory signal peptide to be further prepared.

Procedures of each of the above-mentioned steps may be carried out in accordance with a method known in the genetic engineering field.

A given anaerobic bacterium that is to be transformed is subjected to a method known in the genetic engineering field using the above-mentioned transforming plasmid, thus preparing a transformant.

The present invention further relates to a pharmaceutical composition for an ischemic disease containing the above-mentioned gene transfer carrier.

The pharmaceutical composition of the present invention is not particularly limited as long as it contains the gene transfer carrier of the present invention. With regard to the gene transfer carrier of the present invention, at least one type thereof may be contained, and two or more types thereof may be contained. Furthermore, the pharmaceutical composition of the present invention may be used in a combination with a treatment agent for an ischemic disease or a pharmaceutical composition containing a compound, other than the gene transfer carrier of the present invention, exhibiting a therapeutic effect for an ischemic disease.

Furthermore, the pharmaceutical composition of the present invention may contain an optional component in addition to the gene transfer carrier of the present invention as long as it does not impair the effects of the present invention. Examples of such optional components include pharmaceutically acceptable carriers, excipients, and diluents.

The dosage form of the pharmaceutical composition of the present invention is not particularly limited, and examples thereof include a liquid agent or solid preparation containing the gene transfer carrier of the present invention. The liquid agent may be produced by purifying a liquid culture of the anaerobic bacterium of the gene transfer carrier of the present invention, adding thereto as necessary an appropriate physiological saline, supplementary fluid, or pharmaceutical additive, and charging an ampoule or a vial therewith. The solid preparation may be produced by adding an appropriate protecting agent to a liquid agent, charging an ampoule or a vial therewith, and lyophilizing or L-drying it, or adding an appropriate protecting agent to a liquid agent, lyophilizing or L-drying it, and then charging an ampoule or a vial therewith.

As a method for administering the pharmaceutical composition of the present invention, both oral administration and parenteral administration are possible, but parenteral administration is preferable; for example intravenous injection, subcutaneous injection, local infusion, intracerebroventricular administration, etc. may be carried out, and intravenous injection, that is, systemic administration, is most preferable.

The dose of the gene transfer carrier of the pharmaceutical composition of the present invention is not particularly limited as long as it is a sufficient amount that enables growth at a disease site and an effective therapeutic dose of active protein to be expressed, but from the viewpoint of economy and from the viewpoint of side effects being avoided as far as possible, it is preferable to use as small an amount as possible in a range that can give a necessary therapeutic effect.

The dose of the gene transfer carrier of the pharmaceutical composition of the present invention may be appropriately selected according to the extent of a disease and the body weight, age, and sex of a patient, and may be appropriately increased/decreased according to the degree of improvement.

For example, when the pharmaceutical composition of the present invention is used, the dose is set appropriately according to the therapeutic activity for the disease exhibited by the anaerobic bacterium itself that is used, the type of protein, etc. having therapeutic activity for the disease produced by the anaerobic bacterium used, and the amount of the active protein produced by the anaerobic bacterium used.

Specifically, in the case of for example intravenous administration, since it is particularly necessary to reduce a risk such as embolization by a clump of bacteria, it is preferable to inject an injectable preparation having as low a concentration as possible a plurality of times, or continuously infuse a dilution with an appropriate supplementary fluid. For example, in the case of an adult, 10⁶ to 10¹² cfu of the bacterial cells of the anaerobic bacterium of the present invention per kg of body weight is administered once or multiple times a day for 1 day to multiple days continuously or at appropriate intervals. More specifically, 1 to 1000 mL per adult of a preparation containing 10⁴ to 10¹⁰ cfu/mL of bacterial cells of the *Bifidobacterium* of the present invention is administered directly or after dilution with an appropriate supplementary fluid once or multiple times a day for 1 day to multiple days continuously.

Furthermore, in the case of local administration involving direct administration to the diseased tissue, it is desirable to administer a high concentration injection to multiple positions of the diseased tissue because the bacterium is required to colonize and grow in the entire diseased tissue if possible. For example, in the case of an adult, 10⁶ to 10¹² cfu of bacterial cells of the *Bifidobacterium* of the present invention per kg of body weight is administered once or multiple times a day for 1 day to multiple days as necessary, continuously or at appropriate intervals. More specifically, 1 to 1000 mL per adult of a preparation containing 10⁴ to 10¹⁰ cfu/mL of bacterial cells of the *Bifidobacterium* of the present invention is administered directly, multiple times a day, for 1 day to multiple days continuously as necessary.

When it is confirmed that the bacterium in the diseased tissue has disappeared during the treatment period, the treatment is temporarily suspended, and the bacterium is administered again in the same way as described above.

The expression 'being formed by combining X and Y' in the present invention includes both a case in which X and Y are separate configurations and a case in which X and Y are of the same configuration (for example a configuration containing X and Y). When X and Y are separate configurations, a case in which X and Y further contain another component is also included.

The gene transfer carrier of the present invention colonizes and grows specifically at the site of an ischemic disease and does not grow in normal tissue that is not under an anaerobic environment. Therefore, the gene is delivered specifically to a disease site without there being local administration targeted at the site of an ischemic disease. From the viewpoint of ease of administration and low invasiveness, the pharmaceutical composition of the present invention is therefore preferably administered systemically.

One aspect of the present invention includes a method for diagnosing or treating an ischemic disease using the gene transfer carrier formed from the anaerobic bacterium. In accordance with use of the gene transfer carrier of the present invention, it becomes possible to carry out diagnosis or treatment with high efficiency and high safety.

In the method of the present invention, the gene transfer carrier of the present invention is administered to a target having an ischemic disease. As the administration method, both oral administration and parenteral administration are possible, but parenteral administration is preferable; examples thereof include intravenous injection, subcutaneous injection, local infusion, and intracerebroventricular administration, and intravenous injection, that is, systemic administration, is most preferable.

Therefore, in a preferred embodiment of the method of the present invention, the gene transfer carrier of the present invention is systemically administered. Since the gene transfer carrier of the present invention can specifically deliver the gene to the site of an ischemic disease, even when systemic administration such as intravenous injection is carried out without using a catheter or intramuscular injection, it can colonize and grow at the site of an ischemic disease, and exhibit an effect. Therefore, compared with a conventional therapy, a treatment with very low invasiveness becomes possible.

### [Examples]

The present invention is more specifically explained below through the presentation of selected studies performed to confirm the utility and effectiveness of the invention, however the technical scope of the present invention is not limited to these examples.

### Example 1: Mouse lower limb ischemia model experiment

In order to verify the ability of the gene transfer carrier of the present invention to deliver specifically to the site of an ischemic disease, a mouse model with lower limb ischemia was prepared, and examination was carried out by administering the gene transfer carrier of the present invention.

### (1) Preparation of model

As a test animal, an 8-9 week-old male C57BL/6 mouse (acquired from Charles River Laboratories Japan, Inc.) was used, and a lower limb ischemia model mouse (ischemic model) was prepared by the procedure below. For anesthesia, 0.3 mL of 3.6% chloral hydrate (acquired from Nacalai Tesque, Inc.) was intraperitoneally injected. After the abdomen and the lower limb were shaved, a section from the common femoral artery to the popliteal artery was ligated using 7-0 polypropylene thread, and the femoral artery was excised. After the interior of the wound was washed with physiological saline, the wound was sutured using 5-0 nylon thread.

### (2) Blood flow measurement by laser Doppler blood flow meter

After the mouse was anesthetized, blood flow was measured using a laser Doppler blood flow meter (Moor Instruments) before lower limb muscle removal mentioned in (3). Blood flow was measured from the lower limb to the digit tip on both sides, and expressed as an ischemic side/non-ischemic side ratio. The blood flow measurement was carried out immediately before the model was prepared, immediately thereafter, and 1, 2, 3, 4, 5, 7, 10, 14, 21, and 28 days thereafter.

The results are shown in Figures 1 and 2. In such a lower limb ischemia model, after the surgery the blood flow rate of the ischemic limb became no greater than 20% of that of the non-ischemic limb; it recovered up to about 40% 1 week after the surgery, and it recovered up to about 60% 4 weeks after the surgery (FIG. 1). Change in blood flow was compared between a group to which *B. longum* was administered and a group to which nothing was administered (FIG. 3). There was no significant difference in change in blood flow between the treated group and the non-treated group after surgery (FIG. 2), and it was found that the presence of *B. longum* did not affect the ischemic state.

### (3) Administration and detection of B. longum

*B. longum* that had been transformed by a pBLES100 plasmid and turned into a spectinomycin resistant bacterium was subcultured twice in MRS medium (acquired from Kanto Kagaku). The liquid culture obtained by subculturing twice was subjected to centrifugation using physiological saline and used as a suspension in physiological saline. As a local administration model, immediately after a lower limb ischemia model was prepared, 1 x 10⁹ cfu/mL x 0.1 mL x 2 positions of *B. longum* was administered to each of the ischemic limb and the non-ischemic limb by intramuscular injection. As a systemic administration model, 0.2 mL of 1 × 10⁹ cfu/mL *B. longum* was administered to the tail vein. 1, 2, 3, 4, 5, 7, 10, and 14 days after the bacterium was administered, the left and right lower limb muscles were removed from each of the administration models (n = 5 for each). After the tissue was homogenized, it was smeared on a BL plate and cultured at 37°C under anaerobic conditions for 3 days. After culturing, the number of colonies on each plate was measured to obtain bacterial counts for the ischemic limb and the non-ischemic limb.

The results are shown in Figures 3 through 6. In the case of local administration, accumulation of a larger number of bacteria in the ischemic limb than in the non-ischemic limb was observed at 24 hours after surgery. In the non-ischemic limb, the bacterial count decreased with time thereafter, and almost no bacteria were observed at 168 hours after surgery. This contrasted strongly with the large number of bacteria observed in the ischemic limb at 168 hours. The bacterial count per g of muscle tissue at 168 hours after surgery was 4.8 × 10² for the non-ischemic limb whereas it was 4.1 x 10⁶ for the ischemic limb, there thus being a significant difference (p = 0.039, n = 3)(FIGS. 3 and 4).

Similar results were obtained for the case of systemic administration. That is, in the non-ischemic limb about 0.6 × 10³ bacteria were observed at 24 hours after surgery, decreasing to about 0.1 × 10³ at 48 hours; none were observed at 72 hours after surgery. Conversely, in the ischemic limb about 3.5 × 10⁵ bacteria were observed 24 hours after surgery with the number increasing over time to a maximum of about 2.7 × 10⁶ at 4 days after surgery. Subsequently the bacterial count decreased as the blood flow recovered, and none were observed 10 days after surgery (FIGS. 5 and 6). In the case of systemic administration, it was found that it took longer for the bacteria to colonize compared to local administration, but they specifically colonized the site of the ischemic disease, even with intravenous administration. Since the maximum value for the bacterial count was observed on the 4^{th} day, when the blood flow ratio was the lowest, and henceforth decreased as the blood flow ratio improved, the possibility that *B. longum* grows according to the degree of ischemia is suggested.

### (4) Histological analysis

The lower limb muscle was removed 96 hours after B. *longum* was administered to the mouse in the same way as in (3). The lower limb muscle was immersed in a 20% neutral formalin buffer for 48 hours to fix the tissue. After that, it was embedded in paraffin to form a paraffin block. Paraffin tissue section slides were prepared by slicing the paraffin block into 3 µm sections, which were placed on slide glass, and air dried at 44°C for 24 hours. After deparaffinization, Gram staining was carried out using a neo-B & M Wako Gram staining solution (Wako Pure Chemical Industries, Ltd.). After staining, observation using an optical microscope was carried out.

The results are shown in Figure 6. In the non-ischemic limb no Gram stained bacteria were detected, but in the ischemic limb Gram stained bacteria were observed, and it was confirmed that there was colonization by the administered B. *longum.*

### Example 2: Mouse lower limb necrosis model test

### (1) Preparation of Model

It was suggested in Example 1 that the colonizing B. *longum* count decreased as the blood flow recovered, and in order to confirm this a lower limb necrosis model (necrotic model) mouse with a more serious degree of ischemia was prepared. As a test animal, a 6-7 week-old male BALB/c mouse (acquired from Charles River Laboratories Japan, Inc.) was used. As anesthesia, 0.3 mL of 3.6% chloral hydrate (Nacalai Tesque) was intraperitoneally injected. After the abdomen and the lower limb were shaved, a section from the common femoral artery to the superficial femoral artery was ligated using 7-0 polypropylene thread, and the femoral artery was excised. After the interior of the wound was washed with physiological saline, the wound was sutured using 5-0 nylon thread.

### (2) Blood flow measurement by laser Doppler blood flow meter

Blood flow was measured in the same way as in Example 1; the results are shown in Figure 8. It was found that compared with the ischemic model the timing of recovery of the blood flow was slow for the necrotic model, with the final blood flow rate being about 30% of that of the non-ischemic limb. The level of recovery was about half that of the ischemic model, for which the blood flow recovered to about 60%.

### (3) Administration and detection of B. longum

In the same way as in Example 1, *B*. *longum* was systemically administered, and the muscle tissue of the non-ischemic limb and the ischemic limb was examined. The results are shown in Figures 9 and 10. It was found that compared with the ischemic model, *B. longum* was present in the ischemic limb of the necrotic model for a longer period of time. In the same manner as in the ischemic model, the bacterial count decreased as the blood flow recovered, and no bacteria were observed when the blood flow recovered to some degree. From the above results it was found that, regardless of the duration of the time period, *B. longum* colonized and grew in the ischemic limb as long as the ischemic state continued, and the bacterial count started to decrease as the blood flow recovered (that is, recovery from the ischemic state). Therefore, it can be expected that *B. longum* will be delivered specifically to tissue in an ischemic state, express a gene that has been introduced, have the property of disappearing as the ischemic state recovers, and exhibit excellent effects in the diagnosis and treatment of an ischemic disease.

### Example 3: Safety test

The above-mentioned lower limb ischemia model (ischemic model) mouse and the lower limb necrosis model (necrotic model) mouse were both subjected to a safety test. 2, 3, 4, 6, and 7 days after the bacteria were administered, the kidney, liver, spleen, and heart were removed from each administration model (n = 3 for each). In addition to these organs, in the ischemic model, the lung was removed, and in the necrotic model the blood was removed. After the tissue was homogenized, it was smeared on a BL plate and cultured at 37°C under anaerobic conditions for 3 days. After culturing, the number of colonies on each plate was counted to obtain a bacterial count for each tissue.

The results are shown in Figures 11 and 12. For the ischemic model, *B. longum* was not observed in any of the organs other than the ischemic limb 168 hours after systemic administration. For the necrotic model also, *B. longum* was observed in each organ of the whole body up to 3 days after systemic administration, but it was not observed in organs other than the spleen after 6 days. Furthermore, comparing the treated group and the non-treated group, there was no significant difference in terms of ulceration rate and abscess formation rate of the lower limb, and *B. longum* was not isolated preponderantly from an abscess. From the results above it was found that *B. longum* quickly disappeared from healthy organs and did not have an adverse effect on the disease, such as aggravation of the ischemic state itself or complications.

### Example 4: Miniature swine myocardial infarction model test

As another representative ischemic disease, an ischemic heart disease such as myocardial infarction can be cited, and in recent years an angiogenic treatment, in particular an angiogenic treatment by means of gene transfer, has been attempted for an ischemic heart disease. However, the same problems as those experienced with other ischemic diseases are seen with the treatment of an ischemic heart disease. In an ischemic heart disease in particular, as a local administration method, a very highly invasive method such as arterial injection using a coronary artery catheter, local injection using a coronary artery catheter, or open chest intramyocardial injection is required, and although angiogenic therapy is thought to be beneficial to a target that cannot receive another therapy such as percutaneous coronary intervention (PCI) or a coronary artery bypass graft (CABG) in particular, at the present it is mainly used in combination with other therapies.

In order to confirm that the gene transfer carrier of the present invention functions effectively in myocardial infarction, a test was carried out as follows.

### (1) Preparation of model

As a test animal, a male Gottingen miniature swine (15 kg) (acquired from Chugai Research Institute for Medical Science) was used. 15 mg/kg of Ketaral (acquired from Sankyo) and atropine sulfate (acquired from Tanabeseiyaku) (25 mg) were intramuscularly injected, and an anesthetic state was maintained by an inhalation anesthetic FLO-THENE (acquired from Takeda Chemical Industries, Ltd.) (2% to 5%). After the chest was opened, the left anterior descending coronary artery was ligated using 3-0 silk thread. During the surgery, 2% Xylocaine (acquired from AstraZeneca) (1 mL to 2 mL) was administered. Before closing the chest, the interior of the pericardium was washed with 100 mL of Lactec (acquired from Otsuka Pharmaceutical Co., Ltd.).

### (2) Histological analysis

In order to confirm myocardial infarction, triphenyltetrazolium chloride (TTC) staining, hematoxylin/eosin (HE) staining, and Masson trichrome (MT) staining were carried out. In TTC staining, the heart removed 7 days after preparing the myocardial infarction model was sliced into a thickness of 5 mm and incubated in 1% TTC solution (acquired from SIGMA) at 37°C for 5 minutes. After that, examination was carried out with the naked eye.

In hematoxylin/eosin (HE) staining, after deparaffinization, staining was carried out using hematoxylin for 30 seconds to 1 minute, washing was carried out using running water for 10 minutes, and staining was then carried out with a 0.5% eosin solution for 30 seconds. After staining, dehydration, clearing, and embedding were performed; examination was carried out using an optical microscope.

In Masson's trichrome (MT) staining, each section was Bouin-fixed and then washed with running water for 10 minutes. After washing, the samples were immersed in a mixed solution of equal parts 10% potassium dichromate and 10% trichloroacetic acid for 10 minutes, then immersed in an iron hematoxylin stain solution for 5 minutes, and washed with running water for 5 min. After washing the samples were immersed in a Ponceau/acid fuchsin/azophloxine solution for 1 minute, and then immersed in a 2.5% phosphotungstic acid solution for 10 minutes. Next, the sections were immersed in a 2% orange G solution for 5 minutes and washed with a 1% aqueous solution of acetic acid, stained in a Light Green solution for 3 minutes, and then washed with a 1% aqueous solution of acetic acid. Dehydration, clearing, and embedding were carried out by the same process as in HE staining.

The results are given in Figure 14. In TTC staining, a transmural infarction site was observed in the left ventricular anterior wall and a part of the interventricular septum closer to the anterior wall. Furthermore, in MT and HE staining, myocardial cell depletion and collagen fiber hyperplasia were observed at the same site, and it was confirmed that the myocardial infarction was complete.

### (3) Administration and detection of B. longum

*B. longum* was prepared in the same way as in Example 1; immediately after the myocardial infarction model was prepared 0.1 mL of *B. longum* was intramycocardially administered at a concentration of 1 × 10⁹ cfu/mL to the healthy site and the infarction site at equal intervals of 1 cm (FIG. 13). 4 days and 7 days after the bacteria were administered (n = 2 for each) the heart was removed and separated into the infarction site and the healthy site, homogenized, then smeared on a BL plate, and cultured at 37°C under anaerobic conditions for 3 days. After culturing, the number of colonies on each plate was counted to obtain a bacterial count for the infarction site and the healthy site.

The results are shown in Figure 15. On the 4^{th} day after administration, few bacteria were observed at the healthy site, but significant bacteria were observed at the infarction site. On the 7^{th} day after administration, no bacteria were detected at the healthy site, and bacteria were observed only at the infarction site. It can be seen from the above that in myocardial infarction also *B. longum* colonized and grew specifically at the infarction site.

### Example 5: Constructing a hFGF2 secretory Bifidobacterium.

### (1) Construction of an expression plasmid vector p37.

A plasmid pCDshuttle (International application No: 2009/128272) was used as template for performing PCR using primers CDvecF3 (SEQ ID No: 23) and pUCoriR5 (SEQ ID No: 24) to give a PCR product which was used as a vector. The PCR product amplified from the genomic DNA of Bifidobacterium longum 105A as template with primers Pins37F (SEQ ID No: 25) and Pins37R (SEQ ID No: 26) was used as an insert. PCR was performed in a conventional manner.

The vector and the insert described as above were ligated using the In-Fusion HD Cloning Kit and the Cloning Enhancer (TAKARA BIO, Inc.); this reaction is hereinbelow mentioned as an "In-fusion reaction". A portion of the reaction solution was used for transforming E. coli TOP10 chemically competent cell (Life Technologies Japan Ltd.). The details were according to the product instruction. Transformed E. coli was spread onto an LB agar medium (containing 75µg/mL spectinomycin), statically cultured overnight at 37°C.

Well isolated colonies on the plate as above were picked up, cultured shaken in LB liquid medium (containing 75µg/mL spectinomycin) overnight at 37°C, from which the plasmid DNA was extracted using the QIAprep Spin Miniprep Kit (QIAGEN). A full-length sequence analysis was carried out for this plasmid DNA to confirm the sequence thereof. The complete plasmid was named p37 (SEQ ID No: 39).

### (2) Construction of hFGF2-expressing plasmid (non-secretory).

Fig. 16 shows a summary of constructing the human FGF2-expressing (non-secretory) plasmid pFGF-P37. The vector was prepared as follows: the plasmid p37 was used as template for performing PCR with primers FGF1 (SEQ ID No: 27) and FGF7 (SEQ ID No: 28), amplifying a DNA of approximately 3.8kbp consisting of P37 promoter (SEQ ID No: 29), Hu terminator, pTB6 replicon (plasmid replicon in Bifidobacterium), AAD9 cassette (spectinomycin resistance gene expression unit) and pUCori moiety (plasmid replication origin in E. coli) as the vector. Primers were designed so that the terminal 15 bps of the vector had the same sequence as the terminal 15 bps of the insert shown below. DNA amplification employed the PrimeSTAR HS (Premix) (TAKARA BIO, Inc.), and the PCR conditions were in accordance with the product instruction of the enzyme.

The insert was prepared as follows: based on the amino acid sequence of hFGF2 (a protein of approximately 18kDa whose translation being started from AUG of GenBank Accession No. #NM_002006), a DNA having codons optimized for Bifidobacterium was artificially synthesized (customized by GenScript). Here, the DNA was designed so that a histidine-tag was fused to the C-terminal of the coded hFGF2 protein (SEQ ID No: 30). Using this synthetic DNA as template, PCR amplification with primers FGF3 (SEQ ID No: 31) and FGF4 (SEQ ID No: 32) was carried out in a similar manner as above, amplifying a DNA product of approximately 0.5kbp as the insert.

The vector and insert as above were ligated by an Infusion reaction. The details were in accordance with the product instruction. The In-fusion reaction solution was appropriately diluted with 0.1x TE, of which 2µL was used for transforming E. coli TOP10 chemically competent cell (Life Technologies Japan Ltd.). The details were in accordance with the product instruction. Transformed E. coli was spread onto an LB agar medium (containing 75µg/mL spectinomycin), statically cultured overnight at 37°C.

Well isolated colonies on the plate as above were picked up, cultured shaken in LB liquid medium (containing 75µg/mL spectinomycin) overnight at 37°C, from which the plasmid DNA was extracted using the QIAprep Spin Miniprep Kit (QIAGEN). A part of the plasmid was used for confirming DNA sequence of the hFGF2 expression unit. Sequencing reaction was carried out using the BigDye Terminator v3.1 Cycle Sequencing Kit (Life Technologies Japan Ltd.) and the details were in accordance with the product instruction. Electrophoresis of the sequencing reaction solution was ordered to the Divisions of Instrumental Analysis, Research Center for Human and Environmental Sciences, Shinshu University. Data analysis was carried out using Genetyx Ver.10. The complete plasmid was named pFGF-P37 (SEQ ID No: 33).

### (3) Construction of secretory hFGF2 expression plasmid.

Fig. 16 shows a summary of constructing the human FGF2-expressing (secretory) plasmid pFGF12a. The above-mentioned plasmid pFGF-P37 was used as template for performing PCR with primers FGF35 (SEQ ID No: 34) and FGF26 (SEQ ID No: 35), amplifying a DNA product of approximately 4.2kbp as the vector. Meanwhile, using the genomic DNA of Bifidobacterium longum 105A strain as template, a PCR was performed with primers FGF45 (SEQ ID No: 36) and FGF17 (SEQ ID No: 37), amplifying a DNA product of approximately 0.2kbp as the insert. The insert comprises a secretory signal and well-conserved N-terminal sequence of Sec2 of B. breve UCC2003 (See, Shkoporov AN et. al., Biotechnol Lett (2008) 30: 1983-1988). Amplification of the vector and insert was carried out using PrimeSTAR HS (Premix) (TAKARA BIO, Inc.), where the PCR conditions were in accordance with the product instruction of the enzyme.

The vector and insert as above were ligated by In-fusion reaction, used for transforming E. coli TOP10 in a similar manner to that in constructing above-mentioned FGF expression plasmid (non-secretory), and the DNA sequence of hFGF2 expression unit part of the plasmid was confirmed to give a complete plasmid named pFGF12a (SEQ ID No: 38). The constitution of the hFGF2 expression unit part is as follows:
- P37 promoter: the nucleotides from No. 14 to No. 275 of SEQ ID No: 38;
- the constitution of the hFGF2 coding region: the nucleotides from No. 276 to No. 902 of SEQ ID No: 38;
- the signal sequence: the nucleotides from No. 276 to No. 419 of SEQ ID No: 38;
- the hFGF2 sequence: the nucleotides from No. 420 to No. 881 of SEQ ID No: 38;
- a histidine tag sequence: the nucleotides from No. 882 to No. 899 of SEQ ID No: 38;
- a stop codon: the nucleotides from No. 900 to No. 902 of SEQ ID No: 38;
- Hu terminator: the nucleotides from No. 903 to No. 1016 of SEQ ID No: 38;

The amino acid sequence encoded by said unit is expressed in SEQ ID No: 40, and its constitution is further indicated below.
- the constitution of hFGF2 coding region: the amino acids from No. 1 to No. 208 of SEQ ID No: 40;
- the signal sequence: the amino acids from No. 1 to No. 48 of SEQ ID No: 40 (of which the amino acids from No. 1 to No. 37 corresponds the putative signal peptide region, the amino acids from No. 38 to No. 48 corresponds the putative spacer region, and the putative cleavage site appears between amino acids No. 37 and No. 38);
- the hFGF2 sequence: the amino acids from No. 49 to No. 202 of SEQ ID No: 40;
- a histidine tag sequence: the amino acids from No. 203 to No. 208 of SEQ ID No: 40;

### (4) Preparation of recombinant Bifidobacterium.

Competent cells of the strains Bifidobacterium longum 105A and Bifidobacterium breve JCM1192 were produced according to the methods described in Rossi et al., Letters in Applied Microbiology (1997) 24: 33-36. Namely, said Bifidobacterium were cultured in IMR medium to early logarithmic growth phase, washed with PBS buffer, suspended in KMR buffer and rapidly frozen using liquid nitrogen to give the competent cells. These competent cells were thawed on ice, mixed with the above-mentioned plasmid pFGF12a or pBEshuttle (a mock vector described in International Publication No. WO 2011/093465; no FGF expression), and electroporated using the Gene Pulser II (Bio-Rad Laboratories, Inc.). The electroporation was carried out using a cuvette with 0.2 cm gap under the condition of at 2kV, 25µF and 200Ω. The transformed Bifidobacterium was spread onto an IMR agar medium (comprising 75µg/mL spectinomycin) and cultured at 37°C for 2 days under an anaerobic condition. Well isolated colonies formed on the agar medium were picked up to obtain the recombinant Bifidobacterium.

### (5) Protein expression analysis (Western blotting)

As a medium for culturing Bifidobacterium, 10mL of MRS medium (oxide) was supplemented with 200µL of Vitamin C/cystine solution (containing 350mg/mL of ascorbic acid and 20mg/mL of L-cystine) and 10µL of 75mg/mL of spectinomycin solution. Into this adjusted medium, the above-mentioned recombinant Bifidobacteria, i.e., B. longum 105A/pFGF12a and B. longum 105A/pBEshuttle were inoculated, and cultured at 37°C for 24 hours under an anaerobic condition (an activated culture solution). The activated culture solution was inoculated into a medium which is prepared by adding to said adjusted medium sodium phosphate buffer at final concentration of 166mM, and cultured at 37°C for 18 hours under an anaerobic condition.

Bifidobacterium breve JCM1192/pFGF12a and Bifidobacterium breve JCM1192/pBEshuttle were also cultured in a similar manner as above, except using RCM medium instead of MRS medium. The Vitamin C/cystine solution was not added.

The culture supernatant of the recombinant Bifidobacterium described as above was treated with trichloroacetic acid (TCA) precipitation method according to conventional methods, then redissolved in 1x SDS sample buffer. It was then heated at 95°C for 3 minutes and subjected to Western blot analysis.

The sample described as above was electrophoresed using Mini-PROTEAN® TGX gel (4 to 20%, Bio-Rad) and 1x SDS buffer. An electrophoresis apparatus Mini PROTEAN® Tetra System (Bio-Rad) was used. After being electrophoresed, the gel was transferred onto an iBlot Transfer Stacks using the iBlot Gel Transfer Device. Transferred membrane was washed 3 times in TTBS for 5 minutes each, followed by blocking with 2% blocking solution (Block Ace). After adding a primary antibody Anti FGF-2 human rabbit poly (H-131, Santa Cruz Biotechnology Inc.), the membrane was shaken overnight at 4°C. After the reaction with the primary antibody, the membrane was repeatedly washed 6 times with TTBS for 5 minutes each. After adding a secondary antibody Goat anti-rabbit IgG HRP (Santa Cruz Biotechnology Inc.),the membrane was shaken at room temperature for 3.5 hours. This membrane was repeatedly washed 6 times with TTBS for 5 minutes each, followed by illumination using the illuminating reagent within the ECL Advance Western Blotting Detection Kit (GE Healthcare), detected by an imaging analyzer (Fluor-S MAX, Bio-Rad) (Figs. 17 and 18).

### (6) Protein expression analysis (ELISA)

The culture supernatant was prepared in a similar manner as above up to the step before TCA precipitation, then hFGF2 content was quantified using human FGF2 ELISA kit (Quantikine® ELISAHuman FGF basic Immunoassay, R&D systems, catalogue No: DFB50).

The hFGF2 content in the culture supernatant was 33,058 pg/mL for B. longum 105A/pFGF12a, whereas it was 0 pg/mL for the negative control B. longum 105A/pBEshuttle. On the other hand, it was 11,429 pg/mL for Bifidobacterium breve JCM1192/pFGF12a and 0 pg/mL for its negative control Bifidobacterium breve JCM1192/pBEshuttle.

### Example 6: Evaluating in vivo therapeutic effect in ischemic model mouse

In order to examine the therapeutic effect of the gene transfer carrier of the invention at ischemic disease site, an ischemic model mouse was produced and administered the human FGF2 secretory Bifidobacterium. (1) Preparation of model.

As a test animal, 6- to 8-week-old female Balb/c mice (from CHARLES RIVER LABORATORIES JAPAN, INC.) were used, and the lower limb ischemia model mouse(ischemic model) was prepared using the following procedure. Animals were anesthetized by an intraperitoneal injection of 0.3ml of 3.6% chloral hydrate (from Nacalai Tesque). After epilation of the area from abdomen to lower leg, the common femoral artery, deep femoral artery and superficial femoral artery were ligated with 8-0 polypropylene threads, and the superficial femoral artery was excised. After the interior of the wound was washed with physiological saline, the wound was sutured with a 5-0 nylon thread.

### (2) Blood flow measurement using a laser Doppler blood-flowmeter.

After the mouse was anesthetized, blood flow was measured using a laser Doppler blood flow meter (Moor Instruments). The blood flow was quantified on both sides from lower leg to digit tip, expressed as an ischemic side/non-ischemic side ratio. The blood flow measurement was carried out immediately after producing the model, and at 3 to 4 days, 6 days and 8 days after producing the model.

### (3) Administration of the genetic recombinant B. longum

The human FGF2-secreting Bifidobacterium and control non-secreting Bifidobacterium were cultured for two passes in MRS medium (from KANTO KAGAKU CO.,INC.). The culture solution was then washed by centrifugal washing with physiological saline, suspended in physiological saline and used. On the day after the production of the model, the cells were administered to the orbital sinus at the concentration of 1x 109cfu/ml in 0.2ml, then blood flow in the lower limb was measured as mentioned above (2).

The results are displayed in Figures. 19 and 20, and show the blood flow improving effects of the human FGF2-secreting B. *longum* and the human FGF2-secreting *B. breve,* respectively. In the group treated with the human FGF2-secreting *B. longum,* a significant improvement in blood flow was observed at Day 6 and thereafter as compared to the PBS-treated group (longum FGF12a vs PBS; at Day 6, 0.41 ± 0.11 vs 0.14 ± 0.02; at Day 8, 0.55 ± 0.11 vs 0.32 ± 0.08). In the group treated with the human FGF2-secreting *B. breve,* a significant improvement in blood flow was observed at Day 8 (breve FGF12a vs breve control, 0.67 ± 0.11 vs 0.38 ± 0.02). No significant difference was observed between groups treated with the non FGF-secreting bacterium (control) and with PBS.

### [Industrial Applicability]

In accordance with the gene transfer carrier of the present invention, a gene transfer carrier that colonizes and grows specifically at the site of an ischemic disease and disappears as the disease state improves can be provided. Therefore, compared with a conventional method, an angiogenic therapy can be carried out simply with low invasiveness. Furthermore, since the gene transfer carrier itself is formed from a bacterium, there is no problem with the efficiency with which the gene is transferred to a target site, and a treatment can be carried out with very high efficiency compared with the conventional art. This enables a target such as the elderly, for whom angiogenic therapy should naturally be effective but who cannot be treated because of problems such as high invasiveness and systemic side effects, to be treated effectively.

## Claims

1. A gene transfer carrier consisted of an anaerobic bacterium that can grow specifically at the site of an ischemic disease and can express at least one type of protein that is useful for the diagnosis or treatment of an ischemic disease.

2. The gene transfer carrier according to Claim 1, wherein the anaerobic bacterium is one that has been transformed using a transforming plasmid, said plasmid comprising a DNA sequence coding for the protein that is useful for the diagnosis or treatment of an ischemic disease.

3. The gene transfer carrier according to Claim 1 or 2, wherein the ischemic disease is a chronic ischemic disease.

4. The gene transfer carrier according to any one of Claims 1 to 3, wherein the anaerobic bacterium is a nonpathogenic enterobacterium.

5. The gene transfer carrier according to any one of Claims 1 to 4, wherein the anaerobic bacterium is a *Bifidobacterium.*

6. The gene transfer carrier according to Claim 5, wherein the *Bifidobacterium* is one type selected from the group consisting of *Bifidobacterium adolescentis, Bifidobacterium angulatum, Bifidobacterium animalis, Bifidobacterium asteroides, Bifidobacterium bifidum, Bifidobacterium boum, Bifidobacterium breve, Bifidobacterium catenulatum, Bifidobacterium choerinum, Bifidobacterium coryneforme, Bifidobacterium cuniculi, Bifidobacterium denticolens, Bifidobacterium dentium, Bifidobacterium gallicum, Bifidobacterium gallinarum, Bifidobacterium globosum, Bifidobacterium indicum, Bifidobacterium infantis, Bifidobacterium inopinatum, Bifidobacterium lactis, Bifidobacterium lactentis, Bifidobacterium liberorum, Bifidobacterium longum, Bifidobacterium magnum, Bifidobacterium merycicum, Bifidobacterium minimum, Bifidobacterium mongoliens, Bifidobacterium parvulorum, Bifidobacterium pseudocatenulatum, Bifidobacterium pseudolongum, Bifidobacterium psychraerophilum, Bifidobacterium pullorum, Bifidobacterium ruminale, Bifidobacterium ruminantium, Bifidobacterium saeculare, Bifidobacterium scardovi, Bifidobacterium subtile, Bifidobacterium suis, Bifidobacterium thermacidophillum,* and *Bifidobacterium thermophilum.*

7. The gene transfer carrier according to Claim 6, wherein the *Bifidobacterium* is *Bifidobacterium longum.*

8. The gene transfer carrier according to any one of Claims 1 to 7, wherein the protein that is useful for the diagnosis of an ischemic disease is a fluorescent protein.

9. The gene transfer carrier according to any one of Claims 1 to 7, wherein the protein that is useful for the treatment of an ischemic disease is one type selected from the group consisting of fibroblast growth factor (FGF), endothelial cell growth factor (ECGF), vascular endothelium growth factor (VEGF), hepatocyte growth factor (HGF), angiogenic growth factor (AGF), platelet-derived growth factor (PDGF), transforming growth factor β (TGFβ), a protein having angiogenesis promoting activity such as angiopoietin or ephrin, a factor involved in vasodilation such as a prostaglandin, a colony stimulating factor such as granulocyte colony stimulating factor (G-CSF) or granulocyte-macrophage colony stimulating factor (GM-CSF), nerve growth factor (NGF), brain-derived neurotrophic factor (BDNF), a neurotrophin such as neurotrophin 3, and insulin-like growth factor (IGF).

10. The gene transfer carrier according to any one of Claims 2 to 9, wherein the transforming plasmid is a non-shuttle plasmid.

11. The gene transfer carrier according to any one of Claims 2 to 10, wherein the transforming plasmid further comprises a gene sequence coding for a secretory signal peptide.

12. The gene transfer carrier according to any one of Claims 2 to 11, wherein the transforming plasmid comprises pTB6 rep unit.

13. The gene transfer carrier according to any one of Claims 2 to 12, wherein the transforming plasmid has an expression cassette comprising p37 promoter, HU terminator and a gene coding for FGF2.

14. The gene transfer carrier according to any one of Claims 2 to 13, wherein the transforming plasmid comprises a DNA sequence coding for a polypeptide having the sequence described in SEQ ID No: 40.

15. The gene transfer carrier according to any one of Claims 2 to 14, wherein the transforming plasmid is pFGF12a (SEQ ID No: 38).

16. A pharmaceutical composition for an ischemic disease comprising the gene transfer carrier according to any one of any one of Claims 1 to 15.

17. The pharmaceutical composition according to Claim 16, wherein it is administered by systemic administration.

18. A method for diagnosing or treating an ischemic disease, the method comprising administering an anaerobic bacterium that can specifically grow at the site of an ischemic disease and can express at least one type of protein that is useful for the diagnosis or treatment of an ischemic disease.

19. The method according to Claim 18, wherein said administering is systemically administering.


SEQUENCE LISTING

<110>  Anaeropharma Science

<120>  Therapeutic agent for ischemic diseases

<130>  PCT2527AN

<160>  40

<170>  PatentIn version 3.5

<210>  1
<211>  141
<212>  DNA
<213>  Artificial

<220>
<223>  DNA sequence of signal peptide SP1

<400>  1
atggcggaaa ctaccgttaa gcccacgaag cttgctgtta ttggtgccgg tgccgttggc      60

tccaccctcg ccttcgccgc tgcccagcgt ggcatcgctc gcgagatcgt gcttgaagac     120

atcgccaagg agcgcgtgga a                                               141


<210>  2
<211>  156
<212>  DNA
<213>  Artificial

<220>
<223>  DNA sequence of signal peptide SP2

<400>  2
gtgggtatga ctgagaacgc cgtaaatact gccgtgactt ccacctcctc tcccgcaatt      60

cccgccgaga cttccgccgt ctcccccgca actcgcgcag ccaaccgccc gctgggcaca     120

ccgctgggca agcaccccac ccgcgtgctg tttttg                               156


<210>  3
<211>  165
<212>  DNA
<213>  Artificial

<220>
<223>  DNA sequence of signal peptide SP3

<400>  3
atgttcaata agcgacacat cgtccgtacc attgcggcca ccgccagcat cctggctctg      60

tcgttcaccg cagcctgcgg ttccggccag tccaccgcat ccaattccac cgattcggac     120

gacatcaccc agcagacgta caagccgggc aagctgacca tcgcc                     165


<210>  4
<211>  195
<212>  DNA
<213>  Artificial

<220>
<223>  DNA sequence of signal peptide SP4

<400>  4
atgaccactc acaacagcca gtattccgcc gaaaccgccc atcccgacaa gcaggaaagc      60

agcccggcgc cgaccgccgc cggcaccacg gccagtaacg tctccacaac tggcaacgca     120

accacgccgg acgccagcat cgccctcaac gccgacgcca ctccggtagc cgacgttccc     180

ccgcgtctgt tcggc                                                      195


<210>  5
<211>  177
<212>  DNA
<213>  Artificial

<220>
<223>  DNA sequence of signal peptide SP5

<400>  5
atgaccgcga ttgacgagac cgaccagcgc atcctcacca tgctggaggc cgacggccgc      60

gccacgctcg cgcaactggc ccaggcgacc ggactgtccg tctccgccgc ccagtcgcgc     120

gtgcagaagc tggagaagcg cggcatcatc aagggataca aggccatcat cgaccaa        177


<210>  6
<211>  141
<212>  DNA
<213>  Artificial

<220>
<223>  DNA sequence of signal peptide SP6

<400>  6
atgaagattg cggttgcagg gactggctac gttggattgt ctgtcgcttt gctgctcgct      60

cagcacaatg aagttcatgc actcgacatc attcccgaga aagtcgagca gttaaacaat     120

gggaaaagtc ctattgtcga t                                               141


<210>  7
<211>  126
<212>  DNA
<213>  Artificial

<220>
<223>  DNA sequence of signal peptide SP7

<400>  7
atgtttgcgt gcgtagcccc gtttgcctct gccgattccg cgcagacgag tgctgtggtg      60

tcctcacgtt ctttcccgaa ggcgagttcg gtgaagaaga atttgttcgc cgaatccacc     120

tccacc                                                                126


<210>  8
<211>  204
<212>  DNA
<213>  Artificial

<220>
<223>  DNA sequence of signal peptide SP8

<400>  8
atggttggtg acgacaccgt gacgatatgg tcgattgacg aatcccgaca gccgattcgc      60

cgcgttcgtc tgagtcgatt ccccgctggg gcgcgaatat gccttatcat gggttgcatg     120

acacccgcag agcgtgcgag cgcagtcgca tttgcactca agaactgcgc actggaagcg     180

atgacgccgg gcgaggcaac gatg                                            204


<210>  9
<211>  126
<212>  DNA
<213>  Artificial

<220>
<223>  DNA sequence of signal peptide SP9

<400>  9
atgggcacca tgatgcgaat aggactgacc ggcggcatcg ccgcgggcaa aagtacggtg      60

gcggcgcaac tcaagcaact cggcgcgttg catatcgact acgatgcgct ggcgcatcag     120

atcgtc                                                                126


<210>  10
<211>  153
<212>  DNA
<213>  Artificial

<220>
<223>  DNA sequence of signal peptide SP10

<400>  10
atgatgactg gtgcacaggc cgctcactgt ggttctgtat ccgcaatttc gctgggactg      60

cccgtttcca cggcgattcc cgaagccaaa ggctcactgc cgaaagcctt gttcgtaggc     120

aaagcgccga tttccggcaa actcaagcag cga                                  153


<210>  11
<211>  156
<212>  DNA
<213>  Artificial

<220>
<223>  DNA sequence of signal peptide SP11

<400>  11
atgaagttca ccgttgctaa gaaggccatt gcacttaccg gtgcggttgc catgctgggt      60

tccgttgccg cctgcggttc cgacaccgcc agtggcaagc cggctcaaga taaggacgtt     120

accgaaatca ccgtgtgggc ttgggagccc acgctg                               156


<210>  12
<211>  171
<212>  DNA

<213>  Artificial

<220>
<223>  DNA sequence of signal peptide SP12

<400>  12
atggtgtctt tcaataaact gacccgtact cttgctggca tcgctgccgc cgcgttgatc      60

gttccgctgg ccgcctgtgg tggctccggc aatggtggca ctgccaccgc cgaaggtatc     120

ccggccaagg gcaccgacga tggtaccgag atcaccctgt ggacccgttc c              171


<210>  13
<211>  138
<212>  DNA
<213>  Artificial

<220>
<223>  DNA sequence of signal peptide SP13

<400>  13
atggtcgccg tcctcaggtt cggctgtctg ccatcatgct cggtcttgac tcattattta      60

tccgtgcatg ctgtaggcaa tcggcctgtg gtcggtctcc tcccgcaact tatattgctg     120

tgctgtgcta gcgagtct                                                   138


<210>  14
<211>  186
<212>  DNA
<213>  Artificial

<220>
<223>  DNA sequence of signal peptide SP14

<400>  14
ttgccgggac ctatatgtcc ccggcaacaa cagccgtata tacgctaccg atcagtgctg      60

ccctcatcat ccttggcacc tgccgtggcg gcctcagtct ccttggcgtt ccacccggcg     120

acggcattcc aaccactcca tccgatgacc actagcaagc acagcgagaa gacaatagtg     180

gcccaa                                                                186


<210>  15
<211>  150
<212>  DNA
<213>  Artificial

<220>
<223>  DNA sequence of signal peptide SP15

<400>  15
atgaaacgta gcgattatat gttggcggca ctcgcctcag ccgtcctgcc gaatctgggg      60

gtggccggcg tacgcgagaa cgtgcaggcc agcgcaaccg acgaggccaa aggcatcgat     120

cagaccgtga ttcaagatgc ctcaggcaag                                      150


<210>  16
<211>  195

<212>  DNA
<213>  Artificial

<220>
<223>   DNA sequence of signal peptide SP16

<400>  16
ttggcaagat gggtcactcg gagcgttccg gcaacggcct gtacgtcaac gtgcccggca      60

acaagtacca gccgatcttc gaggccggcg tggaatactt caccgcctga taatcggcgc     120

gtatcgcgtc tgatacggca cacagggaag gaactctcgg gttccttccc ttttttgttc     180

atgccggtca tgggc                                                      195


<210>  17
<211>  195
<212>  DNA
<213>  Artificial

<220>
<223>  DNA sequence of signal peptide SP19

<400>  17
ttggcaagat gggtcactcg gagcgttccg gcaacggcct gtacgtcaac gtgcccggca      60

acaagtacca gccgatcttc gaggccggcg tggaatactt caccgcctga taatcggcgc     120

gtatcgcgtc tgatacggca cacagggaag gaactctcgg gttccttccc ttttttgttc     180

atgccggtca tgggc                                                      195


<210>  18
<211>  240
<212>  DNA
<213>  Artificial

<220>
<223>  DNA sequence of signal peptide SP21

<400>  18
atggcattga ctgatgaaca ggtggagcgg tacgcgcgcc atctgatttt gaagggtgtg      60

ggggtcaaag ggcaaaagcg gttgctggcc tccagcgtgc tcatcatcgg agcgggcggt     120

cttggttctc cggccgccct gtatctggcg gcggccggcg tcggccatat cggactggtg     180

gacggcgatg tggtggatat gagcaatctg caacgccaaa tcatccatac cactgcacgt     240


<210>  19
<211>  240
<212>  DNA
<213>  Artificial

<220>
<223>  DNA sequence of signal peptide SP22

<400>  19
ttggtgtcta tgagaagccc acgcgaggat tttgaggcgg caggcaagcg actgccttgg      60

gatgctgctg ctcgcagtgc agcgctgtcc gccaccgcgc cagtctctga cgtcaaggca     120

tccgccaatg gtgccgacaa tgccagcaac gctgaacatt ccgatgacat gcccaccgtg 180

ccgattcccg cacgcaaggc tgccacgacg ttcgacaccc cctccaagcg tgagcgcatc 240

<210> 20
<211> 180
<212> DNA
<213> Artificial

<220>
<223> DNA sequence of signal peptide SP23

<400> 20
atgaacaagc gatggaacaa actgtgtgtg tccgccctcg cctgcatggc gttggtcgtg 60

ccgttgaccg cctgtgaagg ccaactgccg acgccggctg ctgatacctc caccaaggtt 120

gcgccggatt tgaccgaggc gcaggagaag aagattcgtc tgaagattct caagacgatc 180

<210> 21
<211> 180
<212> DNA
<213> Artificial

<220>
<223> DNA sequence of signal peptide SP24

<400> 21
atggtcggca tgcgcgacgt agccaaagcg gcaggggtgt ccttaagcac cgtttcgttg 60

gtggtcaaca acaccggcta cgtctcggcc gatatgcgtg ccaaagtcga gtccgcgatg 120

cgccagctca actacattcc caacgagctg gcccgcaacc tctaccggaa ccgcaccaac 180

<210> 22
<211> 180
<212> DNA
<213> Artificial

<220>
<223> DNA sequence of signal peptide SP25

<400> 22
gtgatgttat ccacaccctc cactacgttg ttttgcctcg cgctgggcag ccccacttca 60

gcaagagatt gcacagcttg cgttagggtg gagaacatga ctatcacagt atccacagac 120

ggttccgcat tagggaatcc aaacgggcca atgggctggg cctgggccga tcatgagcag 180

<210> 23
<211> 31
<212> DNA
<213> Artificial

<220>
<223> forward primer for amplifying pCDshuttle

<400> 23
tcgaataacg ctttacaaac aattattaac g 31

<210>  24
<211>  33
<212>  DNA
<213>  Artificial

<220>
<223>  reverse primer for pCDshuttle

<400>  24
gacgactgga tccgtagaaa agatcaaagg atc                                    33


<210>  25
<211>  34
<212>  DNA
<213>  Artificial

<220>
<223>  forward primer for p37 insert

<400>  25
tacggatcca gtcgtcctcc caaactttgc agag                                   34


<210>  26
<211>  38
<212>  DNA
<213>  Artificial

<220>
<223>  reverse primer for p37 insert

<400>  26
taaagcgtta ttcgacgccg agactttctt cttgggag                               38


<210>  27
<211>  29
<212>  DNA
<213>  Artificial

<220>
<223>  primer for p37

<400>  27
accatcatca ttgaccttct gctcgtagc                                         29


<210>  28
<211>  32
<212>  DNA
<213>  Artificial

<220>
<223>  primer for p37

<400>  28
cgagccggcg gccatattat ggttcttctt tc                                     32


<210>  29
<211>  262
<212>  DNA
<213>  Bifidobacterium longum

<400>  29
ctcccaaact ttgcagagcc cgaccatcgt ggtcgggctt tttgtgttgt ccgggtggat      60

ttgcataata ggaaagcttg tgtctggaga ggagactcgc tagcacagca cagcaatata     120

agttgcggga ggagaccgac cacaggccga ttgcctacag catgcacgga taaataatga     180

gtcaagaccg agcatgatgg cagacagccg aacctgagga cggcgaccat tcgaaccgcg     240

ttatagaaag aagaaccata at                                              262


<210>  30
<211>  468
<212>  DNA
<213>  Artificial

<220>
<223>  hFGF codon optimized to bifidobacterium

<400>  30
atggccgccg gctcgatcac caccctgccg gccctgccgg aggatggcgg ctcgggcgcc      60

ttcccgccgg gccacttcaa ggacccgaag cgcctgtact gcaagaacgg cggcttcttc     120

ctgcgtatcc acccggacgg ccgtgtggat ggcgtccgtg agaagagcga cccgcatatc     180

aagctgcagc tgcaggccga ggaacgtggc gtggtctcca tcaagggcgt gtgcgccaac     240

cgctacctgg ccatgaagga agacggccgt ctgctggcct cgaagtgcgt caccgatgaa     300

tgcttcttct tcgagcgcct ggaatccaac aactacaaca cctaccgctc ccgtaagtac     360

accagctggt acgtggccct gaagcgtacc ggccagtaca agctgggcag caagaccggc     420

ccgggccaga aggccatcct gttcctgccg atgtccgcca agtcgtga                  468


<210>  31
<211>  43
<212>  DNA
<213>  Artificial

<220>
<223>  reverse primer for hFGF2 insert

<400>  31
gtcaatgatg atggtggtgg tgcgacttgg cggacatcgg cag                        43


<210>  32
<211>  20
<212>  DNA
<213>  Artificial

<220>
<223>  forward primer for hFGF2 insert

<400>  32
atggccgccg gctcgatcac                                                  20


<210>  33
<211>  4236
<212>  DNA

<213>  Artificial

<220>
<223>  hFGF2 expression plasmid

<400>  33
ggatccagtc gtcctcccaa actttgcaga gcccgaccat cgtggtcggg ctttttgtgt      60

tgtccgggtg gatttgcata ataggaaagc ttgtgtctgg agaggagact cgctagcaca     120

gcacagcaat ataagttgcg ggaggagacc gaccacaggc cgattgccta cagcatgcac     180

ggataaataa tgagtcaaga ccgagcatga tggcagacag ccgaacctga ggacggcgac     240

cattcgaacc gcgttataga aagaagaacc ataatatggc cgccggctcg atcaccaccc     300

tgccggccct gccggaggat ggcggctcgg gcgccttccc gccgggccac ttcaaggacc     360

cgaagcgcct gtactgcaag aacggcggct tcttcctgcg tatccacccg gacggccgtg     420

tggatggcgt ccgtgagaag agcgacccgc atatcaagct gcagctgcag gccgaggaac     480

gtggcgtggt ctccatcaag ggcgtgtgcg ccaaccgcta cctggccatg aaggaagacg     540

gccgtctgct ggcctcgaag tgcgtcaccg atgaatgctt cttcttcgag cgcctggaat     600

ccaacaacta caacacctac cgctcccgta agtacaccag ctggtacgtg gccctgaagc     660

gtaccggcca gtacaagctg ggcagcaaga ccggcccggg ccagaaggcc atcctgttcc     720

tgccgatgtc cgccaagtcg caccaccacc atcatcattg accttctgct cgtagcgatt     780

acttcgagca ttactgacga caaagacccc gaccgagatg gtcggggtct ttttgttgtg      840

gtgctgtgac gtgttgtcca accgtattat tccggactag tcctccagga cctcgtctac     900

gaggcgctga gcgaggaatg gcgcaaaagg gacggcgaga tcagcgaccc atgggccaac     960

gacgaggcgg acggatacca gccgccctca tacgagccgg tcaaccccga acgcaggact    1020

ccccagacgc cctccgatgg cctgatctga cgtccgaaaa aaggcgctgt gcgccctttt    1080

taaatctttt ataaatcttt ttacattctt ttagcccctc cgcagcctta ctctcccaac    1140

gggtttcagc cgaaacctac accaaaaggg gagcgaacct acaccaaaag gggagcgaac    1200

ctacaccaaa aggggagcga acctacacca aaaggggagc tatatacacc ttttgttatt    1260

taaggtgcaa gttgtgctat gctgaggcca tgtccaatga gatcgtgaag ttcagcaacc    1320

agttcaacaa cgtcgcgctg aagaagttcg acgccgtgca cctggacgtg ctcatggcga    1380

tcgcctcaag ggtgagggag aagggcacgg ccacggtgga gttctcgttc gaggagctgc    1440

gcggcctcat gcgattgagg aagaacctga ccaacaagca gctggccgac aagatcgtgc    1500

agacgaacgc gcgcctgctg gcgctgaact acatgttcga ggattcgggc aagatcatcc    1560

agttcgcgct gttcacgaag ttcgtcaccg acccgcagga ggcgactctc gcggttgggg    1620

tcaacgagga gttcgcgttc ctgctcaacg acctgaccag ccagttcacg cgcttcgagc    1680

tggccgagtt cgccgacctc aagagcaagt acgccaagga gttctaccgc agggccaagc    1740

agtaccgcag ctccggaatc tggaagatcg gccgcgacga gttctgccga ctgcttggcg    1800

ttccaccgtc ggcaataacc cagacacgat atctgaatca gaaggttctt cagccaattc 1860

aggaggagtg tgggcctctc cttggcctga agatcgagcg ccagtacgtg aaacgcaggc 1920

tgtcgggctt cgtgttcaca ttcgcccgcg agacccctcc ggtgatcgac gccaggcccg 1980

tggaggcgag gaagacggac ggcgacggca agggccattg gacgagcgtt gccgggtacg 2040

gcgaggtgtt cacgaccacg gcgttgttcg acgtgacggc cgcccgggct cacttcgacg 2100

gcaccgttga agccggggag tgccgtttct gcgcgtttga cgcgcgcaac cgcgaacatc 2160

atgcgcggaa cgccggaagg ctgttctagc ggccgtgtcc gcgcctctgg ggcggttgcg 2220

cctgccatgg gtcgatctgc cgctgttcgg cctcacgctg gtctgtgcgc tgcctgatct 2280

ccctgagcag gtcggccttg gtcctggggg cgcttcgctc ctcgaacggg ccgctctccc 2340

ccaggtcctc gggctcgctc aggtccaacg gctcgtcacc ggacggctcg ggccggttct 2400

ctccctgtgc cgggttctcc gcctgtgcgc gttgttcggc catgcgcagt gcgagggcct 2460

tcacctgttc ggggcttgtc gactcgattt tcgttcgtga atacatgtta taataactat 2520

aactaataac gtaacgtgac tggcaagaga tatttttaaa acaatgaata ggtttacact 2580

tactttagtt ttatggaaat gaaagatcat atcatatata atctagaata aaattaacta 2640

aaataattat tatctagata aaaaatttag aagccaatga aatctataaa taaactaaat 2700

taagtttatt taattaacaa ctatggatat aaaataggta ctaatcaaaa tagtgaggag 2760

gatatatttg aatacatacg aacaaattaa taaagtgaaa aaaatacttc ggaaacattt 2820

aaaaaataac cttattggta cttacatgtt tggatcagga gttgagagtg gactaaaacc 2880

aaatagtgat cttgactttt tagtcgtcgt atctgaacca ttgacagatc aaagtaaaga 2940

aatacttata caaaaaatta gacctatttc aaaaaaaata ggagataaaa gcaacttacg 3000

atatattgaa ttaacaatta ttattcagca agaaatggta ccgtggaatc atcctcccaa 3060

acaagaattt atttatggag aatggttaca agagctttat gaacaaggat acattcctca 3120

gaaggaatta aattcagatt taaccataat gctttaccaa gcaaaacgaa aaaataaaag 3180

aatatacgga aattatgact tagaggaatt actacctgat attccatttt ctgatgtgag 3240

aagagccatt atggattcgt cagaggaatt aatagataat tatcaggatg atgaaaccaa 3300

ctctatatta actttatgcc gtatgatttt aactatggac acgggtaaaa tcataccaaa 3360

agatattgcg ggaaatgcag tggctgaatc ttctccatta gaacataggg agagaatttt 3420

gttagcagtt cgtagttatc ttggagagaa tattgaatgg actaatgaaa atgtaaattt 3480

aactataaac tatttaaata acagattaaa aaaattataa aaaaattgaa aaaatggtgg 3540

aaacactttt ttcaattttt ttagatcttg agcaaaaggc cagcaaaagg ccaggaaccg 3600

taaaaaggcc gcgttgctgg cgtttttcca taggctccgc ccccctgacg agcatcacaa 3660

aaatcgacgc tcaagtcaga ggtggcgaaa cccgacagga ctataaagat accaggcgtt 3720

tccccctgga agctccctcg tgcgctctcc tgttccgacc ctgccgctta ccggatacct 3780

gtccgccttt ctcccttcgg gaagcgtggc gctttctcat agctcacgct gtaggtatct 3840

cagttcggtg taggtcgttc gctccaagct gggctgtgtg cacgaacccc ccgttcagcc 3900

cgaccgctgc gccttatccg gtaactatcg tcttgagtcc aacccggtaa gacacgactt 3960

atcgccactg gcagcagcca ctggtaacag gattagcaga gcgaggtatg taggcggtgc 4020

tacagagttc ttgaagtggt ggcctaacta cggctacact agaagaacag tatttggtat 4080

ctgcgctctg ctgaagccag ttaccttcgg aaaaagagtt ggtagctctt gatccggcaa 4140

acaaaccacc gctggtagcg gtggtttttt tgtttgcaag cagcagatta cgcgcagaaa 4200

aaaaggatct caagaagatc ctttgatctt ttctac 4236

<210> 34
<211> 24
<212> DNA
<213> Artificial

<220>
<223> primer for pFGF-P37

<400> 34
gccgccggct cgatcaccac cctg 24

<210> 35
<211> 28
<212> DNA
<213> Artificial

<220>
<223> primer for pFGF-P37

<400> 35
catattatgg ttcttctttc tataacgc 28

<210> 36
<211> 39
<212> DNA
<213> Artificial

<220>
<223> forward primer for signal peptide

<400> 36
aagaaccata atatggagca catgaagatg ttccggcac 39

<210> 37
<211> 33
<212> DNA
<213> Artificial

<220>
<223> reverse primer for signal peptide

<400> 37
gatcgagccg gcggccaatg ccacccaatc cgg 33

<210>  38
<211>  4377
<212>  DNA
<213>  Artificial

<220>
<223>  transformation plasmid pFGF12a

<400>  38
ggatccagtc gtcctcccaa actttgcaga gcccgaccat cgtggtcggg ctttttgtgt      60

tgtccgggtg gatttgcata ataggaaagc ttgtgtctgg agaggagact cgctagcaca      120

gcacagcaat ataagttgcg ggaggagacc gaccacaggc cgattgccta cagcatgcac      180

ggataaataa tgagtcaaga ccgagcatga tggcagacag ccgaacctga ggacggcgac      240

cattcgaacc gcgttataga aagaagaacc ataatatgga gcacatgaag atgttccggc      300

acctatcctc cgtttttgct attgcgacca ttgcgccgct ggcgttggcg gccacgctag      360

ccgtgacgcc tgcaatcgca caggccgacc agctgcccaa cccggattgg gtggcattgg      420

ccgccggctc gatcaccacc ctgccggccc tgccggagga tggcggctcg ggcgccttcc      480

cgccgggcca cttcaaggac ccgaagcgcc tgtactgcaa gaacggcggc ttcttcctgc      540

gtatccaccc ggacggccgt gtggatggcg tccgtgagaa gagcgacccg catatcaagc      600

tgcagctgca ggccgaggaa cgtggcgtgg tctccatcaa gggcgtgtgc gccaaccgct      660

acctggccat gaaggaagac ggccgtctgc tggcctcgaa gtgcgtcacc gatgaatgct      720

tcttcttcga gcgcctggaa tccaacaact acaacaccta ccgctcccgt aagtacacca      780

gctggtacgt ggccctgaag cgtaccggcc agtacaagct gggcagcaag accggcccgg      840

gccagaaggc catcctgttc ctgccgatgt ccgccaagtc gcaccaccac catcatcatt      900

gaccttctgc tcgtagcgat tacttcgagc attactgacg acaaagaccc cgaccgagat      960

ggtcggggtc tttttgttgt ggtgctgtga cgtgttgtcc aaccgtatta ttccggacta      1020

gtcctccagg acctcgtcta cgaggcgctg agcgaggaat ggcgcaaaag ggacggcgag      1080

atcagcgacc catgggccaa cgacgaggcg gacggatacc agccgccctc atacgagccg      1140

gtcaaccccg aacgcaggac tccccagacg ccctccgatg gcctgatctg acgtccgaaa      1200

aaaggcgctg tgcgcccttt ttaaatcttt tataaatctt tttacattct tttagcccct      1260

ccgcagcctt actctcccaa cgggtttcag ccgaaaccta caccaaaagg ggagcgaacc      1320

tacaccaaaa ggggagcgaa cctacaccaa aaggggagcg aacctacacc aaaaggggag      1380

ctatatacac cttttgttat ttaaggtgca agttgtgcta tgctgaggcc atgtccaatg      1440

agatcgtgaa gttcagcaac cagttcaaca acgtcgcgct gaagaagttc gacgccgtgc      1500

acctggacgt gctcatggcg atcgcctcaa gggtgaggga gaagggcacg gccacggtgg      1560

agttctcgtt cgaggagctg cgcggcctca tgcgattgag gaagaacctg accaacaagc      1620

agctggccga caagatcgtg cagacgaacg cgcgcctgct ggcgctgaac tacatgttcg 1680

aggattcggg caagatcatc cagttcgcgc tgttcacgaa gttcgtcacc gacccgcagg 1740

aggcgactct cgcggttggg gtcaacgagg agttcgcgtt cctgctcaac gacctgacca 1800

gccagttcac gcgcttcgag ctggccgagt tcgccgacct caagagcaag tacgccaagg 1860

agttctaccg cagggccaag cagtaccgca gctccggaat ctggaagatc ggccgcgacg 1920

agttctgccg actgcttggc gttccaccgt cggcaataac ccagacacga tatctgaatc 1980

agaaggttct tcagccaatt caggaggagt gtgggcctct ccttggcctg aagatcgagc 2040

gccagtacgt gaaacgcagg ctgtcgggct tcgtgttcac attcgcccgc gagacccctc 2100

cggtgatcga cgccaggccc gtggaggcga ggaagacgga cggcgacggc aagggccatt 2160

ggacgagcgt tgccgggtac ggcgaggtgt tcacgaccac ggcgttgttc gacgtgacgg 2220

ccgcccgggc tcacttcgac ggcaccgttg aagccgggga gtgccgtttc tgcgcgtttg 2280

acgcgcgcaa ccgcgaacat catgcgcgga acgccggaag gctgttctag cggccgtgtc 2340

cgcgcctctg gggcggttgc gcctgccatg ggtcgatctg ccgctgttcg gcctcacgct 2400

ggtctgtgcg ctgcctgatc tccctgagca ggtcggcctt ggtcctgggg gcgcttcgct 2460

cctcgaacgg gccgctctcc cccaggtcct cgggctcgct caggtccaac ggctcgtcac 2520

cggacggctc gggccggttc tctccctgtg ccgggttctc cgcctgtgcg cgttgttcgg 2580

ccatgcgcag tgcgagggcc ttcacctgtt cggggcttgt cgactcgatt ttcgttcgtg 2640

aatacatgtt ataataacta taactaataa cgtaacgtga ctggcaagag atatttttaa 2700

aacaatgaat aggtttacac ttactttagt tttatggaaa tgaaagatca tatcatatat 2760

aatctagaat aaaattaact aaaataatta ttatctagat aaaaaattta gaagccaatg 2820

aaatctataa ataaactaaa ttaagtttat ttaattaaca actatggata taaaataggt 2880

actaatcaaa atagtgagga ggatatattt gaatacatac gaacaaatta ataaagtgaa 2940

aaaaatactt cggaaacatt taaaaaataa ccttattggt acttacatgt ttggatcagg 3000

agttgagagt ggactaaaac caaatagtga tcttgacttt ttagtcgtcg tatctgaacc 3060

attgacagat caaagtaaag aaatacttat acaaaaaatt agacctattt caaaaaaaat 3120

aggagataaa agcaacttac gatatattga attaacaatt attattcagc aagaaatggt 3180

accgtggaat catcctccca aacaagaatt tatttatgga gaatggttac aagagcttta 3240

tgaacaagga tacattcctc agaaggaatt aaattcagat ttaaccataa tgctttacca 3300

agcaaaacga aaaaataaaa gaatatacgg aaattatgac ttagaggaat tactacctga 3360

tattccattt tctgatgtga gaagagccat tatggattcg tcagaggaat taatagataa 3420

ttatcaggat gatgaaacca actctatatt aactttatgc cgtatgattt taactatgga 3480

cacgggtaaa atcataccaa aagatattgc gggaaatgca gtggctgaat cttctccatt 3540

agaacatagg gagagaattt tgttagcagt tcgtagttat cttggagaga atattgaatg 3600

gactaatgaa aatgtaaatt taactataaa ctatttaaat aacagattaa aaaaattata 3660

aaaaaattga aaaaatggtg gaaacacttt tttcaatttt tttagatctt gagcaaaagg 3720

ccagcaaaag gccaggaacc gtaaaaaggc cgcgttgctg gcgtttttcc ataggctccg 3780

cccccctgac gagcatcaca aaaatcgacg ctcaagtcag aggtggcgaa acccgacagg 3840

actataaaga taccaggcgt ttccccctgg aagctccctc gtgcgctctc ctgttccgac 3900

cctgccgctt accggatacc tgtccgcctt tctcccttcg ggaagcgtgg cgctttctca 3960

tagctcacgc tgtaggtatc tcagttcggt gtaggtcgtt cgctccaagc tgggctgtgt 4020

gcacgaaccc cccgttcagc ccgaccgctg cgccttatcc ggtaactatc gtcttgagtc 4080

caacccggta agacacgact tatcgccact ggcagcagcc actggtaaca ggattagcag 4140

agcgaggtat gtaggcggtg ctacagagtt cttgaagtgg tggcctaact acggctacac 4200

tagaagaaca gtatttggta tctgcgctct gctgaagcca gttaccttcg gaaaaagagt 4260

tggtagctct tgatccggca aacaaaccac cgctggtagc ggtggttttt ttgtttgcaa 4320

gcagcagatt acgcgcagaa aaaaaggatc tcaagaagat cctttgatct tttctac 4377

<210> 39
<211> 5058
<212> DNA
<213> Artificial

<220>
<223> p37 plasmid

<400> 39
ggatccagtc gtcctcccaa actttgcaga gcccgaccat cgtggtcggg ctttttgtgt 60

tgtccgggtg gatttgcata ataggaaagc ttgtgtctgg agaggagact cgctagcaca 120

gcacagcaat ataagttgcg ggaggagacc gaccacaggc cgattgccta cagcatgcac 180

ggataaataa tgagtcaaga ccgagcatga tggcagacag ccgaacctga ggacggcgac 240

cattcgaacc gcgttataga aagaagaacc ataatatggc tcccaagaag aaagtctcgg 300

cgtcgaataa cgctttacaa acaattatta acgcccggtt accaggcgaa gaggggctgt 360

ggcagattca tctgcaggac ggaaaaatca gcgccattga tgcgcaatcc ggcgtgatgc 420

ccataactga aaacagcctg gatgccgaac aaggtttagt tataccgccg tttgtggagc 480

cacatattca cctggacacc acgcaaaccg ccggacaacc gaactggaat cagtccggca 540

cgctgtttga aggcattgaa cgctgggccg agcgcaaagc gttattaacc catgacgatg 600

tgaaacaacg cgcatggcaa acgctgaaat ggcagattgc caacggcatt cagcatgtgc 660

gtacccatgt cgatgtttcg gatgcaacgc taactgcgct gaaagcaatg ctggaagtga 720

agcaggaagt cgcgccgtgg attgatctgc aaatcgtcgc cttccctcag gaagggattt 780

tgtcgtatcc caacggtgaa gcgttgctgg aagaggcgtt acgcttaggg gcagatgtag 840

tgggggcgat tccgcatttt gaatttaccc gtgaatacgg cgtggagtcg ctgcataaaa 900

ccttcgccct ggcgcaaaaa tacgaccgtc tcatcgacgt tcactgtgat gagatcgatg 960

acgagcagtc gcgctttgtc gaaaccgttg ctgccctggc gcaccatgaa ggcatgggcg 1020

cgcgagtcac cgccagccac accacggcaa tgcactccta taacggggcg tatacctcac 1080

gcctgttccg cttgctgaaa atgtccggta ttaactttgt cgccaacccg ctggtcaata 1140

ttcatctgca aggacgtttc gatacgtatc caaaacgtcg cggcatcacg cgcgttaaag 1200

agatgctgga gtccggcatt aacgtctgct ttggtcacga tgctgtcttc gatccgtggt 1260

atccgctggg aacggcgaat atgctgcaag tgctgcatat ggggctgcat gtttgccagt 1320

tgatgggcta cgggcagatt aacgatggcc tgaatttaat cacccaccac agcgcaagga 1380

cgttgaattt gcaggattac ggcattgccg ccggaaacag cgccaacctg attatcctgc 1440

cggctgaaaa tgggtttgat gcgctgcgcc gtcaggttcc ggtacgttat tcggtacgtg 1500

gcggcaaggt gattgccagc acacaaccgg cacaaaccac cgtatatctg gagcagccag 1560

aagccatcga ttacaaacgt tgaccttctg ctcgtagcga ttacttcgag cattactgac 1620

gacaaagacc ccgaccgaga tggtcggggt ctttttgttg tggtgctgtg acgtgttgtc 1680

caaccgtatt attccggact agtcctccag gacctcgtct acgaggcgct gagcgaggaa 1740

tggcgcaaaa gggacggcga gatcagcgac ccatgggcca acgacgaggc ggacggatac 1800

cagccgccct catacgagcc ggtcaacccc gaacgcagga ctccccagac gccctccgat 1860

ggcctgatct gacgtccgaa aaaaggcgct gtgcgccctt tttaaatctt ttataaatct 1920

ttttacattc ttttagcccc tccgcagcct tactctccca acgggtttca gccgaaacct 1980

acaccaaaag gggagcgaac ctacaccaaa aggggagcga acctacacca aaaggggagc 2040

gaacctacac caaaagggga gctatataca ccttttgtta tttaaggtgc aagttgtgct 2100

atgctgaggc catgtccaat gagatcgtga agttcagcaa ccagttcaac aacgtcgcgc 2160

tgaagaagtt cgacgccgtg cacctggacg tgctcatggc gatcgcctca agggtgaggg 2220

agaagggcac ggccacggtg gagttctcgt tcgaggagct gcgcggcctc atgcgattga 2280

ggaagaacct gaccaacaag cagctggccg acaagatcgt gcagacgaac gcgcgcctgc 2340

tggcgctgaa ctacatgttc gaggattcgg gcaagatcat ccagttcgcg ctgttcacga 2400

agttcgtcac cgacccgcag gaggcgactc tcgcggttgg ggtcaacgag gagttcgcgt 2460

tcctgctcaa cgacctgacc agccagttca cgcgcttcga gctggccgag ttcgccgacc 2520

tcaagagcaa gtacgccaag gagttctacc gcagggccaa gcagtaccgc agctccggaa 2580

tctggaagat cggccgcgac gagttctgcc gactgcttgg cgttccaccg tcggcaataa 2640

cccagacacg atatctgaat cagaaggttc ttcagccaat tcaggaggag tgtgggcctc 2700

tccttggcct gaagatcgag cgccagtacg tgaaacgcag gctgtcgggc ttcgtgttca 2760

cattcgcccg cgagacccct ccggtgatcg acgccaggcc cgtggaggcg aggaagacgg 2820

acggcgacgg caagggccat tggacgagcg ttgccgggta cggcgaggtg ttcacgacca 2880

cggcgttgtt cgacgtgacg gccgcccggg ctcacttcga cggcaccgtt gaagccgggg 2940

agtgccgttt ctgcgcgttt gacgcgcgca accgcgaaca tcatgcgcgg aacgccggaa 3000

ggctgttcta gcggccgtgt ccgcgcctct ggggcggttg cgcctgccat gggtcgatct 3060

gccgctgttc ggcctcacgc tggtctgtgc gctgcctgat ctccctgagc aggtcggcct 3120

tggtcctggg ggcgcttcgc tcctcgaacg ggccgctctc ccccaggtcc tcgggctcgc 3180

tcaggtccaa cggctcgtca ccggacggct cgggccggtt ctctccctgt gccgggttct 3240

ccgcctgtgc gcgttgttcg gccatgcgca gtgcgagggc cttcacctgt tcggggcttg 3300

tcgactcgat tttcgttcgt gaatacatgt tataataact ataactaata acgtaacgtg 3360

actggcaaga gatattttta aaacaatgaa taggtttaca cttactttag ttttatggaa 3420

atgaaagatc atatcatata taatctagaa taaaattaac taaaataatt attatctaga 3480

taaaaaattt agaagccaat gaaatctata aataaactaa attaagttta tttaattaac 3540

aactatggat ataaaatagg tactaatcaa aatagtgagg aggatatatt tgaatacata 3600

cgaacaaatt aataaagtga aaaaaatact tcggaaacat ttaaaaaata accttattgg 3660

tacttacatg tttggatcag gagttgagag tggactaaaa ccaaatagtg atcttgactt 3720

tttagtcgtc gtatctgaac cattgacaga tcaaagtaaa gaaatactta tacaaaaaat 3780

tagacctatt tcaaaaaaaa taggagataa aagcaactta cgatatattg aattaacaat 3840

tattattcag caagaaatgg taccgtggaa tcatcctccc aaacaagaat ttatttatgg 3900

agaatggtta caagagcttt atgaacaagg atacattcct cagaaggaat taaattcaga 3960

tttaaccata atgctttacc aagcaaaacg aaaaaataaa agaatatacg gaaattatga 4020

cttagaggaa ttactacctg atattccatt ttctgatgtg agaagagcca ttatggattc 4080

gtcagaggaa ttaatagata attatcagga tgatgaaacc aactctatat taactttatg 4140

ccgtatgatt ttaactatgg acacgggtaa aatcatacca aaagatattg cgggaaatgc 4200

agtggctgaa tcttctccat tagaacatag ggagagaatt ttgttagcag ttcgtagtta 4260

tcttggagag aatattgaat ggactaatga aaatgtaaat ttaactataa actatttaaa 4320

taacagatta aaaaaattat aaaaaaattg aaaaaatggt ggaaacactt ttttcaattt 4380

ttttagatct tgagcaaaag gccagcaaaa ggccaggaac cgtaaaaagg ccgcgttgct 4440

ggcgtttttc cataggctcc gcccccctga cgagcatcac aaaaatcgac gctcaagtca 4500

gaggtggcga aacccgacag gactataaag ataccaggcg tttccccctg gaagctccct 4560

cgtgcgctct cctgttccga ccctgccgct taccggatac ctgtccgcct ttctcccttc 4620

gggaagcgtg gcgctttctc atagctcacg ctgtaggtat ctcagttcgg tgtaggtcgt 4680

tcgctccaag ctgggctgtg tgcacgaacc ccccgttcag cccgaccgct gcgccttatc 4740

cggtaactat cgtcttgagt ccaacccggt aagacacgac ttatcgccac tggcagcagc 4800

cactggtaac aggattagca gagcgaggta tgtaggcggt gctacagagt tcttgaagtg 4860

gtggcctaac tacggctaca ctagaagaac agtatttggt atctgcgctc tgctgaagcc 4920

agttaccttc ggaaaaagag ttggtagctc ttgatccggc aaacaaacca ccgctggtag 4980

cggtggtttt tttgtttgca agcagcagat tacgcgcaga aaaaaaggat ctcaagaaga 5040

tcctttgatc ttttctac 5058

<210> 40
<211> 208
<212> PRT
<213> Artificial

<220>
<223> polypeptide coded by pFGF12a

<400> 40

Met Glu His Met Lys Met Phe Arg His Leu Ser Ser Val Phe Ala Ile
1               5                   10                  15

Ala Thr Ile Ala Pro Leu Ala Leu Ala Ala Thr Leu Ala Val Thr Pro
            20                  25                  30

Ala Ile Ala Gln Ala Asp Gln Leu Pro Asn Pro Asp Trp Val Ala Leu
        35                  40                  45

Ala Ala Gly Ser Ile Thr Thr Leu Pro Ala Leu Pro Glu Asp Gly Gly
    50                  55                  60

Ser Gly Ala Phe Pro Pro Gly His Phe Lys Asp Pro Lys Arg Leu Tyr
65                  70                  75                  80

Cys Lys Asn Gly Gly Phe Phe Leu Arg Ile His Pro Asp Gly Arg Val
                85                  90                  95

Asp Gly Val Arg Glu Lys Ser Asp Pro His Ile Lys Leu Gln Leu Gln
            100                 105                 110

Ala Glu Glu Arg Gly Val Val Ser Ile Lys Gly Val Cys Ala Asn Arg
        115                 120                 125

Tyr Leu Ala Met Lys Glu Asp Gly Arg Leu Leu Ala Ser Lys Cys Val
    130                 135                 140

Thr Asp Glu Cys Phe Phe Phe Glu Arg Leu Glu Ser Asn Asn Tyr Asn
145                 150                 155                 160

Thr Tyr Arg Ser Arg Lys Tyr Thr Ser Trp Tyr Val Ala Leu Lys Arg
                165                 170                 175

Thr Gly Gln Tyr Lys Leu Gly Ser Lys Thr Gly Pro Gly Gln Lys Ala
            180                 185                 190

Ile Leu Phe Leu Pro Met Ser Ala Lys Ser His His His His His His
        195                 200                 205